# FASCICULE DE BREVET EUROPEEN

(11) **EP 2 220 062 B1**
(45) Date de publication et mention de la délivrance du brevet: **17.08.2016**
(21) Numéro de dépôt: 08851666.1
(22) Date de dépôt: 20.11.2008
(51) Int. Cl.: C07D 295/135, C07D 295/205, A61K 31/4453, A61K 31/496, C07C 243/32, A61P 31/12

(54) **NOUVEAUX INHIBITEURS DU VIRUS DU PAPILLOME HUMAIN ET LES COMPOSITIONS PHARMACEUTIQUES LES CONTENANT**
NEUE INHIBITOREN DES HUMANEN PAPILLOMAVIRUS UND PHARMAZEUTISCHE ZUSAMMENSETZUNGEN, DIE DIESE ENTHALTEN
NEW HUMAN PAPILLOMA VIRUS INHIBITORS AND PHARMACEUTICAL COMPOSITIONS CONTAINING SAME

(30) Priorité: 20.11.2007 FR 0759175
(43) Date de publication de la demande: 25.08.2010
(73) Titulaire: Anaconda Pharma, 94800 Villejuif (FR)
(72) Inventeur: BLUMENFELD, Marta, F-75013 Paris (FR); COMPERE, Delphine, F-92330 Sceaux (FR); GAUTHIER, Jean-Michel, F-78700 Conflans-Sainte-Honorine (FR)
(74) Mandataire: Regimbeau
(86) Numéro de dépôt international: PCT/EP2008/065915
(87) Numéro de publication internationale: WO 2009/065893

(56) Documents cités:
- WO-A-2004/108673
- WO-A-2007/135106
- DE-A1- 3 723 232
- WERMUTH C G: "MOLECULAR VARIATIONS BASED ON ISOSTERIC REPLACEMENTS" PRACTICE OF MEDICINAL CHEMISTRY, XX, XX, 1 janvier 1996 (1996-01-01), pages 203-237, XP002190259

## Description

La présente invention concerne des nouveaux composés antiviraux dirigés contre le virus du papillome, les compositions pharmaceutiques les contenant, leur procédé de préparation, ainsi que leur utilisation pour le traitement ou la prévention d'une infection par le virus du papillome.

Les virus du papillome sont des virus non enveloppés dont le génome est formé par un ADN double brin d'environ 8 kb. Ils sont très répandus dans la nature et provoquent des lésions épithéliales chez l'Homme ainsi que chez de nombreux animaux dont le lapin, le cheval, le chien et l'espèce bovine. Près d'une centaine de virus du papillome humains (VPH) ont été décrits. Ils sont classés en fonction de leurs sites d'infection. Environ 30 VPH ont été isolés à partir de muqueuses anogénitales (col de l'utérus, vagin, vulve, pénis, anus, rectum). Les autres VPH sont associés à des lésions cutanées. Les VPH à tropisme cutané incluent, entre autres, VPH1, VPH2, VPH3, VPH4, VPH5, VPH7, VPH8, VPH9, VPH10, VPH12, VPH14, VPH15, VPH17, VPH19, VPH20, VPH21, VPH22, VPH23, VPH24, VPH25, VPH26, VPH27, VPH28, VPH29, VPH38, VPH41, VPH47, VPH49. Ils sont associés à des lésions comme des verrues (de type vulgaire, plantaire, myrmécie, superficielle, plate...) et des maladies comme l'épidermo-dysplasie verruciforme.

Les VPH de type mucogénital sont impliqués dans des maladies laryngées et anogénitales dont certains cancers. Ils sont souvent classés en VPH à haut risque et VPH à bas risque, en se référant au type de lésions auxquels ils sont associés. Les VPH à bas risque incluent, entre autres, VPH6, VPH11, VPH13, VPH32, VPH34, VPH40, VPH42, VPH43, VPH44, VPH53, VPH54, VPH55, VPH57, VPH58, VPH74, VPH91. Les VPH à bas risque sont associés à des lésions bénignes comme les condylomes (verrues génitales telles les condylomes acuminés et condylomes plans), les papillomes laryngés, conjonctivaux ou buccaux ou d'autres lésions épithéliales comme des néoplasies intra-épithéliales de bas grade ou des papillomatoses récurrentes respiratoires, et plus rarement à des papuloses bowénoïdes ou des néoplasies intra-épithéliales de grade élevé ou des carcinomes. Les VPH à haut risque incluent, entre autres, VPH16, VPH18, VPH31, VPH33, VPH35, VPH39, VPH45, VPH51, VPH52, VPH56, VPH59, VPH61, VPH62, VPH66, VPH67, VPH68, VPH72. Ils sont impliqués dans des lésions intra-épithéliales de bas grade qui peuvent évoluer vers des lésions de plus haut grade allant jusqu'à des cancers, en particulier le cancer du col de l'utérus et autres cancers ano-génitaux.

Les infections génitales par des VPH sont les infections sexuellement transmises les plus fréquentes dans le monde, y compris dans les pays développés, avec plus de 20 millions de personnes infectées aux Etats-Unis. La prévalence des infections aux VPH varie de 3 à 42 % selon les pays et affecte 10 à 20 % de la population sexuellement active dans les pays industrialisés. Dans une partie de cette population, l'infection persiste et peut conduire à des cancers dans le cas des VPH à haut risque.

On estime à 1 à 2 % la prévalence des verrues génitales (condylomes) dans la population sexuellement active des pays industrialisés, soit environ 3 500 000 nouveaux cas par an dans ces pays et 28 000 000 dans le monde. Les verrues génitales peuvent être trouvées sur des parties du corps comprenant ou périphériques de l'anus, la vulve, le vagin, le col de l'utérus et le pénis.

Les traitements des verrues génitales reposent sur plusieurs stratégies, depuis la destruction physique (cryothérapie, laser CO₂, électro-chirurgie, excision chirurgicale), l'application d'agents cytotoxiques (TCA, podophylline, podofilox) jusqu'à l'application d'agents immuno-modulateurs (interféron, imiquimod, polyphenon E). Cependant, aucune de ces méthodes n'élimine complètement toutes les particules virales et des taux de récurrence importants, accompagnés d'effets secondaires sérieux sont observés avec les stratégies thérapeutiques actuelles. Ceci renforce un besoin en nouvelles stratégies pour contrôler ou éliminer les infections par les virus du papillome.

La demande internationale WO 2004/108673 décrit des inhibiteurs du virus du papillome de formules (I) et (II) suivantes : 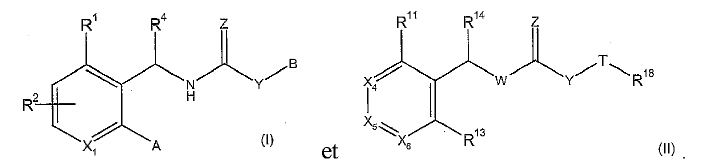 Toutefois, il n'y a toujours pas, à ce jour, de traitement antiviral ciblant spécifiquement les pathogènes viraux que sont les virus du papillome, contrairement à ce qui existe dans le traitement d'autres maladies virales, comme celles causées par VIH, les virus de l'Herpès ou influenza.

Les virus du papillome infectent les épithéliums pluristratifiés et leur cycle viral est étroitement lié à l'organogenèse de ces organes et à la différentiation des kératinocytes. Après infection, le génome viral est présent et répliqué en faible nombre dans les cellules basales de l'épithélium. A mesure que les cellules se différencient, l'expression des gènes viraux et le nombre de copies du génome viral augmentent, jusqu'à l'expression des gènes de la capside virale et la formation de virions infectieux dans les kératinocytes totalement différenciés.

Le génome des VPH code potentiellement pour une dizaine de protéines. Les protéines les plus précocement exprimées, E1 et E2, sont impliquées dans la réplication du génome viral et la régulation de l'expression des gènes viraux. Les autres protéines précoces de ces virus (E4, E5, E6, E7) ont des fonctions en relation avec la prolifération cellulaire ou des rôles non encore complètement élucidés. L'existence des protéines E3 et E8 est encore incertaine. Les protéines tardives L1 et L2 sont celles qui forment la capside virale.

Les 2 seules protéines virales nécessaires et suffisantes pour la réplication des VPH sont E1 et E2. Elles sont capables de former un complexe E1/E2 et de se fixer sur l'origine de réplication (Ori) des VPH, une séquence contenue dans le génome viral et portant des sites proches reconnus par E1 et par E2. E2 est capable de se fixer avec une très grande affinité sur les sites E2 alors que E1, seule, ne possède pas une très grande affinité pour les sites E1. L'interaction entre E1 et E2 augmente la fixation de E1 sur l'Ori par coopérativité de fixation à l'ADN. Une fois fixée à l'ADN, E1 n'interagit plus avec E2 puis forme un hexamère. Les activités hélicase et ATPase de E1 permettent le déroulement de l'ADN viral qui est ensuite répliqué par la machinerie cellulaire de réplication. L'interaction entre les protéines virales E1 et E2 est absolument nécessaire à la réplication des VPH dans les cellules. La disruption de l'interaction entre E1 et E2 aboutit à une absence de réplication virale.

Les inventeurs ont cherché à mettre au point des petites molécules qui inhibent la réplication des VPH, de préférence à bas risque, en interférant notamment avec la formation du complexe entre les protéines E1 et E2.

Une solution a été trouvée par l'élaboration de nouveaux dérivés.

La présente invention a pour objet ces nouveaux dérivés, leur synthèse, ainsi que leur utilisation dans des compositions pharmaceutiques aptes à être utilisées dans la prévention et le traitement des pathologies liées à une inhibition de la réplication des VPH, comme, à titre d'exemples, VPH1, VPH2, VPH3, VPH4, VPH5, VPH7, VPH8, VPH9, VPH10, VPH12, VPH14, VPH15, VPH17, VPH19, VPH20, VPH21, VPH22, VPH23, VPH24, VPH25, VPH26, VPH27, VPH28, VPH29, VPH38, VPH41, VPH47, VPH49, VPH6, VPH11, VPH13, VPH32, VPH34, VPH40, VPH42, VPH43, VPH44, VPH53, VPH54, VPH55, VPH57, VPH58, VPH74, VPH91, VPH16, VPH18, VPH31, VPH33, VPH35, VPH39, VPH45, VPH51, VPH52, VPH56, VPH59, VPH61, VPH62, VPH66, VPH67, VPH68, VPH72 de préférence les VPH à bas risque tels que VPH6, VPH11, VPH13, VPH32, VPH34, VPH40, VPH42, VPH43, VPH44, VPH53, VPH54, VPH55, VPH57, VPH58, VPH74, VPH91.

Les nouveaux dérivés objets de la présente invention sont actifs contre le virus du papillome. Ils sont aussi capables d'inhiber l'interaction E1/E2.

Dans le cadre de la présente invention, on donne les définitions suivantes :
"Alkyle" ou "Alk" signifie une chaîne hydrocarbonée saturée, linéaire ou ramifiée, monovalente ou divalente, comprenant de 1 à 6 atomes de carbone tel que le groupement méthyle, éthyle, propyle, isopropyle, tert-butyle, méthylène, éthylène, propylène, ...

"Acyle" signifie un groupement -CORₐ où Rₐ est un groupement alkyle tel que défini précédemment ou phényle, par exemple acétyle, éthylcarbonyle, benzoyle, ...

"Acylamino" signifie un groupement -NHC(O)R où R est un groupement alkyle tel que défini précédemment.

"Acylaminoalkyle" signifie un groupement -AlkNHC(O)R_{b} où Alk et R_{b} sont des groupements alkyle tels que définis précédemment.

"Alkoxy" signifie un groupement -OAlk où Alk est un groupement alkyle tel que défini précédemment. Alkoxy comprend par exemple méthoxy, éthoxy, *n*-propyloxy, *tert*-butyloxy, ...

"Aryle" signifie un système monocyclique ou bicyclique aromatique comprenant de 4 à 10 atomes de carbone, étant compris que dans le cas d'un système bicyclique, l'un des cycles présente un caractère aromatique et l'autre cycle est aromatique ou insaturé. Aryle comprend par exemple les groupements phényle, naphthyle, indényle, benzocyclobutényle, ...

"Hétérocycle" signifie un système monocyclique ou bicyclique fusionné, spiro-fusionné ou ponté, de 3 à 12 chaînons, saturé, insaturé ou aromatique, comprenant de 1 à 4 hétéroatomes, identiques ou différents, choisis parmi oxygène, soufre et azote, et contenant éventuellement 1 ou 2 groupements oxo (=O) ou thioxo (=S), étant compris que dans le cas d'un système bicyclique, l'un des cycles peut présenter un caractère aromatique et l'autre cycle est aromatique ou insaturé. Hétérocycle comprend par exemple les groupements pipéridyle, pipérazyle, furyle, thiényle, pyrrolyle, pyrazolyle, imidazolyle, pyridyle, pyrimydile, pyrazynile, pyradizinyle, benzofuryle, benzothiényle, indolyle, quinolyle, isoquinolyle, benzodioxolyle, benzodioxinyle, benzo[1,2,5]thiadiazolyle, benzo[1,2,5]oxadiazolyle, [1,2,3]triazolyle, [1,2,4]triazolyle,

"Alkylthio" signifie un groupement -SAlk où Alk est un groupement alkyle tel que défini précédemment. Alkylthio comprend par exemple méthylthio, éthylthio, isopropylthio, heptylthio, ...

"Arylalkyle" signifie un groupement -Alk-Ar où Alk représente un groupement alkyle tel que défini précédemment et Ar représente un groupement aryle tel que défini précédemment.

"Atome d'halogène" signifie un atome de fluor, de brome, de chlore ou d'iode.

"Cycloalkyle" signifie un système monocyclique ou polycyclique tel que bicyclique fusionné ou ponté, saturé, comprenant de 3 à 12 atomes de carbone tel que le groupement cyclopropyle, cyclobutyle, cyclopentyle, cyclohexyle, cycloheptyle, cyclooctyle, adamantyle, décalinyle, norbornyle, ...

"Cycloalkényle" signifie un système monocyclique ou polycyclique tel que bicyclique fusionné ou ponté, insaturé, comprenant de 3 à 12 atomes de carbone tel que le groupement cyclopropényle, cyclobutényle, cyclopentényle, cyclohexényle, ...

"Monoalkylamino" signifie un groupement -NHAlk où Alk est un groupement alkyle tel que défini précédemment.

"Dialkylamino" signifie un groupement -NAlkAlk' où Alk et Alk' représentent chacun indépendamment l'un de l'autre un groupement alkyle tel que défini précédemment.

"Monoalkylamide" signifie un groupement -C(O)NHAlk où Alk est un groupement alkyle tel que défini précédemment.

"Dialkylamide" signifie un groupement -C(O)NAlkAlk' où Alk et Alk' représentent chacun l'un indépendamment de l'autre un groupement alkyle tel que défini précédemment.

"N-cycloalkyle" signifie un radical cycloalkyle tel que défini précédemment, comprenant un atome d'azote, relié au restant de la molécule par cet atome. N-cycloalkyle comprend par exemple le groupement pipérid-1-yle ou pyrrolid-1-yle. "N-cycloalkényle" signifie un radical cycloalkényle tel que défini précédemment, comprenant un atome d'azote, relié au restant de la molécule par cet atome. N-cycloalkényle comprend par exemple le groupement tétrahydropyridin-1-yle.

"Ester" signifie un groupement -C(O)OR_{c} avec R_{c} représentant un groupement alkyle tel que défini précédemment.

"Halogénoalkyle" signifie une chaîne hydrocarbonée saturée, linéaire ou ramifiée, comprenant de 1 à 6 atomes de carbone et substituée par un ou plusieurs et notamment 1 à 6 atomes d'halogène tel que le groupement trifluorométhyle, 2,2,2-trifluoroéthyle, ...

"Halogénoalkoxy" signifie une chaîne hydrocarbonée saturée, linéaire ou ramifiée, comprenant de 1 à 6 atomes de carbone et substituée par un ou plusieurs et notamment 1 à 6 atomes d'halogène, ladite chaîne étant reliée au composé par un atome d'oxygène tel que le groupement
trifluorométhoxy, 2,2,2-trifluoroéthoxy, ...

"Halogénoalkylthio" signifie une chaîne hydrocarbonée saturée, linéaire ou ramifiée, comprenant de 1 à 6 atomes de carbone et substituée par un ou plusieurs et notamment 1 à 6 atomes d'halogène, ladite chaîne étant rattachée par un atome de soufre tel que le groupement trifluorométhylthio, ...

« Groupe protecteur » ou « groupe de protection » signifie le groupe qui bloque sélectivement le site réactif dans un composé multifonctionnel de telle sorte qu'une réaction chimique peut être effectuée sélectivement au niveau d'un autre site réactif non protégé dans la signification classiquement associée à celui-ci en chimie de synthèse.

« Isomérisme » signifie des composés qui ont des formules moléculaires identiques mais qui diffèrent par nature ou dans la séquence de liaison de leurs atomes ou dans l'agencement de leurs atomes dans l'espace. Les isomères qui diffèrent dans l'agencement de leurs atomes dans l'espace sont désignés par « stéréoisomères ». Les stéréoisomères qui ne sont pas des images dans un miroir l'un de l'autre sont désignés par « diastéréoisomères », et les stéréoisomères qui sont des images dans un miroir non superposables sont désignés par « énantiomères » ou isomères optiques. Les « stéréoisomères » se réfèrent aux racémates, énantiomères et diastéréoisomères.

« Pharmaceutiquement acceptable » signifie celui qui est de manière générale sûr, non toxique, et qui n'est pas indésirable biologiquement, aussi bien pour une utilisation vétérinaire que pour une utilisation pharmaceutique humaine.

« Sels pharmaceutiquement acceptables » d'un composé signifie des sels qui sont pharmaceutiquement acceptables, tels que définis ici, et qui possèdent l'activité pharmacologique souhaitée du composé parent. Il devrait être compris que toutes les références aux sels pharmaceutiquement acceptables comprennent les formes d'addition de solvants (solvates) ou les formes cristallines (polymorphes) tels que définis ici, du même sel d'addition d'acide ou de base. Une revue des sels pharmaceutiquement acceptables est notamment décrite *dans* J. Phare. Sci., 1977, 66, 1-19.

Les « acides pharmaceutiquement acceptables » signifient les sels d'acides non toxiques issus d'acides organiques ou minéraux. Parmi les acides pharmaceutiquement acceptables, on peut citer à titre non limitatif les acides chlorhydrique, bromhydrique, sulfurique, phosphonique, nitrique, acétique, trifluoroacétique, lactique, pyruvique, malonique, succinique, glutarique, fumarique, tartrique, maléïque, citrique, ascorbique, oxalique, méthane sulfonique, camphorique, benzoïque, toluènesulfonique, ...

Les « bases pharmaceutiquement acceptables » signifient les sels basiques non toxiques issues de bases organiques ou minérales, formés quand un proton acide présent dans le composé parent est remplacé par un ion métallique ou est coordiné à une base organique. Parmi les bases pharmaceutiquement acceptables, on peut citer à titre non limitatif l'hydroxyde de sodium, l'hydroxyde de potassium, l'hydroxyde de lithium, l'hydroxyde de calcium, la triétylamine, la tertbutylamine, la 2-diéthylaminoéthanol, l'éthanolamine, l'éthylènediamine, la dibenzyléthylènediamine, la pipéridine, la pyrrolidine, la morpholine, la pipérazine, la benzylamine, l'arginine, la lysine, l'histidine, la glucosamine, les hydroxydes d'ammonium quaternaire, ...

Dans la présente demande de brevet, les composés chimiques sont nommés selon la nomenclature IUPAC (The International Union of Pure and Applied Chemistry) lorsque celle-ci peut s'appliquer audit composé.

La présente invention a pour objet les composés de formule (I) : 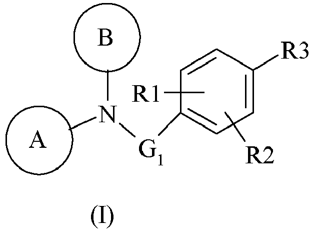 ainsi que leurs stéréoisomères, dans laquelle :
**G₁** représente un groupement 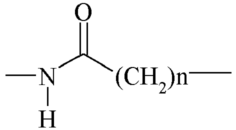 où n est un entier compris entre 1 et 4, de préférence n vaut 1,
**R1** représente un groupement alkoxy, tel que méthoxy, de préférence en position ortho par rapport à R3,
**R2** représente un atome d'halogène, tel que le brome, de préférence en position méta par rapport à R3,
**R3** représente :
   - un groupement 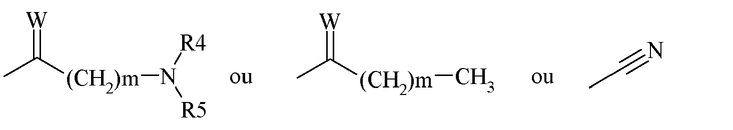 dans lequel :
      - **W** représente un atome d'oxygène, ou NH,
      - **m** est un entier compris entre 0 et 2, et
      - **R4** et **R5** représentent indépendamment l'un de l'autre un atome d'hydrogène, un groupement alkyle linéaire ou ramifié ou un groupement alkoxy,
   - ou le groupements suivants : 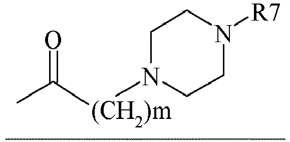 dans lesquels :
      - **R7** un groupe alkyle, tel que méthyle,
      - **m** est un entier compris entre 0 et 2,

**A** représente un groupement aryle, tel que phényle, substitué, de préférence en position para, par un groupement alkoxy, tel que méthoxy, ou acyle, tel qu'acétyle, **B** représente un groupement aryl, de préférence un phényle,
- substitué en position ortho par un hétérocycle, de préférence un N-cycloalkyle, tel qu'un groupement pipéridine, et
- éventuellement substitué en position ortho' par un groupement alkyle, tel qu'un méthyle.

La présente invention concerne également les sels pharmaceutiquement acceptables des composés de formule (I).

Un groupe de composés préférés de formule (I) est celui où : **R3** représente :
- un groupement : 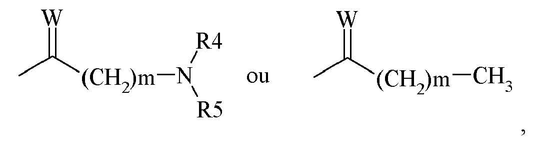 dans lequel :
   - **W** représente un atome d'oxygène ou NH,
   - **m** est un entier compris entre 0 et 2, et
   - **R4** et **R5** représentent indépendamment l'un de l'autre un atome d'hydrogène, un groupement alkyle linéaire ou ramifié, tel qu'un méthyle ou un tertiobutyle, ou un groupement alkoxy, tel qu'un méthoxy,
- ou le groupement : 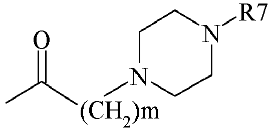 dans lequel R7 représente un groupe alkyle, tel que méthyle, et m représente un entiers compris entre 0 et 2,
   et **R1, R2, A** et **B** sont tels que définis précédemment. Un groupe de composés de formule (I) particulièrement préférés est celui où : **R1** représente un groupement méthoxy en position ortho par rapport à R3, **R2** représente un atome de brome en position méta par rapport à R3, **R3** représente :
- un groupement : 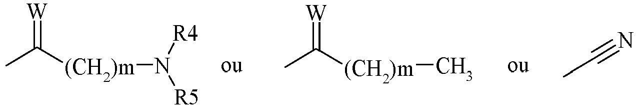 et avantageusement 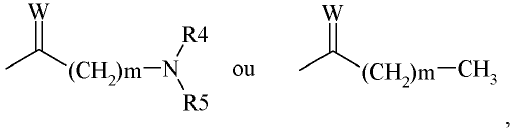 dans lequel :
   - **W** représente un atome d'oxygène ou NH,
   - **m** est un entier compris entre 0 et 2, et
   - **R4** et **R5** représentent indépendamment l'un de l'autre un atome d'hydrogène, un groupement alkyle linéaire ou ramifié, tel qu'un méthyle ou un tertiobutyle, ou un groupement alkoxy,
- ou le groupement : 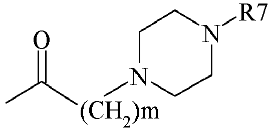 dans lequel R7 est représente un groupe alkyle, tel que méthyle, et m est un entier compris entre 0 et 2,
   **A** représente un groupement phényle substitué en position para par un groupement méthoxy, ou acétyle, et
   **B** représente un groupement phényle substitué en position ortho par un groupement pipéridine et substitué en position ortho' par un groupement méthyle.

Les composés encore plus particulièrement préférés sont rassemblés dans le
tableau I :

**Tableau I**

| | |
|---|---|
| **1** | 5-Bromo-2-méthoxy-4-[*N*'-(4-méthoxy-phényl)-*N*'-(2-méthyl-6-pipéridin-1-yl-phényl)-hydrazinocarbonylméthyl]-benzamide |
| **2** | 5-Bromo-2-méthoxy-4-[*N*'-(4-méthoxy-phényl)-*N'*-(2-méthyl-6-pipéridin-1-yl-phényl)-hydrazinocarbonylméthyl]-*N,N-*diméthyl-benzamide |
| **3** | [2-Bromo-5-méthoxy-4-(4-méthyl-pipérazine-1-carbonyl)-phényl]-acétic acid *N'*-(4-méthoxy-phényl)-*N'*-(2-méthyl-6-pipéridin-1-yl-phényl)-hydrazide |
| **4** | 4-[*N'*-(4-Acétyl-phényl)-*N'*-(2-méthyl-6-pipéridin-1-yl-phényl)-hydrazinocarbonylméthyl]-5-bromo-2-méthoxy-benzamide |
| **5** | (4-Acétyl-2-bromo-5-méthoxy-phényl)-acétic acid *N'*-(4-méthoxy-phényl)-*N'*-(2-méthyl-6-pipéridin-1-yl-phényl)-hydrazide |
| **6** | 5-Bromo-2-*N*-diméthoxy-4-[*N'*-(4-méthoxy-phényl)-*N'*-(2-méthyl-6-pipéridin-1-yl-phényl)-hydrazinocarbonylméthyl]-*N*-méthyl-benzamide |
| **7** | 5-Bromo-*N*-*tert*-butyl-2-méthoxy-4-[*N*'-(4-méthoxy-phényl)-*N*'-(2-méthyl-6-pipéridin-1-yl-phényl)-hydrazinocarbonylméthyl]-benzamide |
| **8** | (2-Bromo-4-cyano-5-méthoxy-phényl)-acétic acid *N*'-(4-méthoxy-phényl)-*N*'-(2-méthyl-6-pipéridin-1-yl-phényl)-hydrazide |

La présente invention a également pour objet les compositions pharmaceutiques comprenant au moins un composé de formule (I) ou un sel pharmaceutiquement acceptable de celui-ci, en association avec un excipient pharmaceutiquement acceptable.

Les compositions pharmaceutiques selon l'invention peuvent être des compositions administrables dans l'organisme par toute voie d'administration. De manière non-exhaustive, la voie d'administration des compositions pharmaceutiques selon l'invention peut être topique, entérale ou parentérale, de préférence une administration buccale, conjonctivale, cutanée, endotrachéale, intradermale, intraépidermale, intramusculaire, intravasculaire, laryngale, nasale, ophtalmique, orale, rectale, respiratoire, sous-cutanée, transcutanée ou vaginale. Il est généralement avantageux de formuler de telles compositions pharmaceutiques sous forme de dose unitaire. Chaque dose comprend alors une quantité prédéterminée du principe actif, associée au véhicule, excipients et/ou adjuvants appropriés, calculée pour obtenir un effet thérapeutique donné. A titre d'exemple de forme de dose unitaire administrable par voie orale on peut citer les comprimés, les gélules, les granules, les poudres et les solutions ou suspensions orales. A titre d'exemple de forme de dose unitaire administrable par voie topique (notamment pour le traitement local des verrues génitales et péri-anales externes), on peut citer les ovules, gels, crèmes, lotions, solutions et patchs.

Les formulations appropriées pour la forme d'administration choisie sont connues et décrites, par exemple dans le Remington, The Science and Practice of Pharmacy, 19ième édition, 1995, Mack Publishing Company et peuvent donc être facilement préparées par l'homme de l'art.

Il est connu que la posologie varie d'un individu à l'autre, selon la nature et l'intensité de l'affection, la voie d'administration choisie, le poids, l'âge et le sexe du malade en conséquence les doses efficaces devront être déterminées en fonction de ces paramètres par le spécialiste en la matière. A titre indicatif, les doses efficaces pourraient s'échelonner entre 1 et 500 mg par jour.

La présente invention a également pour objet un composé de formule (I) ou un sel pharmaceutiquement acceptable de celui-ci tel que défini précédemment pour son utilisation comme médicament.

La présente invention a également pour objet l'utilisation des composés de formule (I) ou un sel pharmaceutiquement acceptable de celui-ci pour le traitement ou la prévention d'une infection par le virus du papillome, chez l'Homme de préférence.

La présente invention a également pour objet l'utilisation des composés de formule (I) ou de leurs sels pharmaceutiquement acceptables pour inhiber la réplication du virus du papillome par inhibition de la formation du complexe protéinique E1/E2.

La présente invention a en outre pour objet l'utilisation des composés de formule (I) ou de leurs sels pharmaceutiquement acceptables pour la préparation d'un médicament destiné au traitement ou à la prévention d'une infection par le virus du papillome, chez l'Homme de préférence.

La présente invention a en particulier pour objet l'utilisation des composés de formule (I) ou de leurs sels pharmaceutiquement acceptables pour la préparation d'un médicament destiné au traitement ou à la prévention d'une infection par un virus du papillome à bas risque, tel que VPH6, VPH7, VPH11, VPH13, VPH32, VPH34, VPH40, VPH42, VPH43, VPH44, VPH53, VPH54, VPH55, VPH57, VPH58, VPH74, VPH91.

La présente invention a en particulier pour objet l'utilisation des composés de formule (I) ou de leurs sels pharmaceutiquement acceptables pour la préparation d'un médicament destiné au traitement ou à la prévention d'une infection par VPH6 et/ou VPH11.

Ainsi, la présente invention a également pour objet l'utilisation des composés de formule (I) ou de leurs sels pharmaceutiquement acceptables pour la préparation d'un médicament destiné au traitement ou à la prévention des lésions et maladies associées aux infections par le virus de papillome.

La présente invention a en particulier pour objet l'utilisation des composés de formule (I) ou de leurs sels pharmaceutiquement acceptables pour la préparation d'un médicament destiné au traitement ou à la prévention des verrues ano-génitales, comme les condylomes acuminés et condylomes plans, les papillomes laryngés, conjonctivaux ou buccaux et d'autres lésions épithéliales, comme des papillomatoses récurrentes respiratoires et des néoplasies intra-épithéliales de bas grade et de haut grade, des papuloses bowénoïdes, des verrues (vulgaires, plantaires, myrmécies, superficielles, plates...), des épidermodysplasies verruciformes, des carcinomes, en particulier ano-génitaux, et toutes les lésions qui sont associées au virus du papillome.

La présente invention a en particulier pour objet l'utilisation des composés de formule (I) ou de leurs sels pharmaceutiquement acceptables pour la préparation d'un médicament destiné au traitement ou à la prévention des verrues ano-génitales, comme les condylomes acuminés et condylomes plans, les papillomes laryngés, conjonctivaux ou buccaux et d'autres lésions épithéliales, comme des papillomatoses récurrentes respiratoires et des néoplasies intra-épithéliales de bas grade et toutes les lésions qui sont associées au virus du papillome.

Les composés objets de la présente invention peuvent être préparés selon la voie de synthèse décrite ci-après, en utilisant des précurseurs de formule (II) et (III) suivantes : 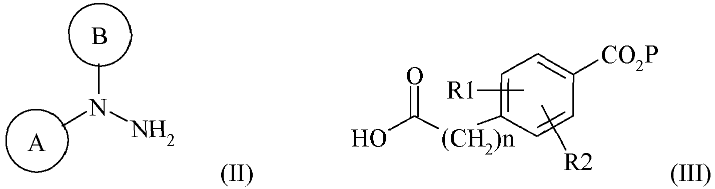 dans lesquelles n, A, B, R1 et R2 sont tels que définis précédemment et P représente un groupe protecteur de fonction acide, tel qu'un groupement (C₁-C₄)alkyle linéaire ou ramifié.

Selon cette voie de synthèse, on effectue un couplage peptidique entre les composés (II) et (III) en présence par exemple d'EDCI dans un milieu basique et polaire pour conduire au composé de formule (IV) :

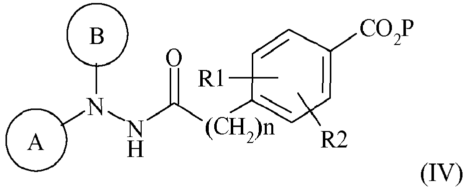

Puis on déprotège le groupement -CO₂P du composé de formule (IV) par hydrolyse, pour obtenir le composé de formule (V) suivante :

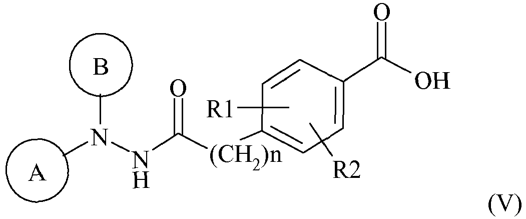

Dans le cas où dans le groupement correspondant à R3, m = 0, les composés de formule (I) peuvent être obtenus en faisant réagir l'amine diversement substituée terminale de R3 soit directement sur la fonction acide du composé de formule (V), soit sur l'une de ses formes activées, obtenue par exemple par réaction du composé de formule (V) avec le *N*-hydroxysuccinimide ou avec le chlorure d'oxalyle.

Dans le cas où dans le groupement correspondant à R3, m ≠ 0, les composés de formule (I) peuvent être obtenus en passant par l'intermédiaire éther d'énol à partir de l'ester correspondant de formule (IV) en utilisant par exemple le réactif de Tebbe. Cet intermédiaire éther d'énol soumis ensuite à une réaction de Mannich, avec par exemple du paraformaldéhyde en présence de diméthylamine dans un solvant polaire, conduit au produit de formule (I) dans le cas où m ≠ 0.

Les composés de formule (I) peuvent également être obtenus à partir des précurseurs de formule (II) et (X) suivantes : 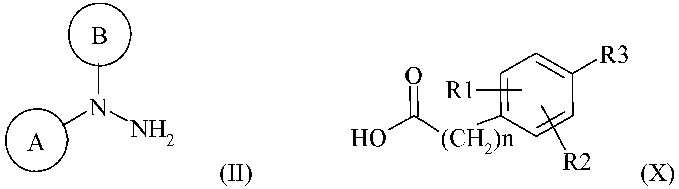 dans lesquelles, n, A, B, R1, R2 et R3 sont tels que définis précédemment.

Dans ce cas, on effectue un couplage peptidique entre les composés (II) et (X) en présence par exemple d'EDCI dans un milieu basique et polaire pour conduire au composé de formule (I) tel que précédemment décrit.

Le précurseur de formule (II) peut être obtenu à partir du composé de formule (VI) suivante: 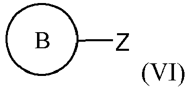 dans laquelle B est tel que défini précédemment et Z est un halogène tel que fluor ou brome.

Dans le cas où Z est un fluor, on soumet le composé de formule (VI) à une substitution nucléophile aromatique en milieu basique et polaire en présence du composé de formule (VII) suivante : 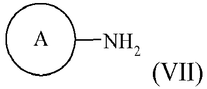 dans laquelle A est tel que défini précédemment, pour obtenir le composé de formule (VIII) suivante: 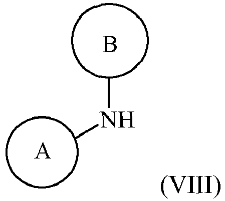 dans laquelle A et B sont tels que définis précédemment.

Dans le cas où Z est un brome, on soumet le composé de formule (VI) à une réaction de Buchwald en présence de bis(2-diphénylphosphinophényl)éther et d'un catalyseur tel que le tris(dibenzylidèneacétone)dipalladium (0) en milieu basique et apolaire en présence du composé (VII) tel que défini précédemment pour conduire au composé de formule (VIII) tel que défini précédemment.

Le composé de formule (VIII) est mis en présence de nitrite de sodium en milieu acide puis réduit par un hydrure, par exemple l'hydrure de lithium et d'aluminium, pour donner le composé de formule (II) tel que défini précédemment.

Dans le cas où R2 représente un atome d'hydrogène, le composé de formule (III) peut être obtenu selon les méthodes de la littérature (J. Med. Chem. 1998, 41, 5219 ou WO 0135900).

Dans le cas où R2 représente un atome de brome, le composé de formule (III) peut être obtenu en faisant réagir du dibrome en milieu acide avec un précurseur de formule (IX) suivante: 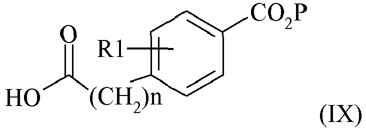 dans laquelle R1, n et P sont tels que définis précédemment.

Les composés de formule (VI) et (VII) sont soit des composés commerciaux, soit des composés obtenus selon des méthodes connues de la synthèse organique aisément accessibles et compréhensibles à l'homme du métier.

Dans le cas préféré où B est un phényle substitué par une pipéridine, on peut préparer les composés de formule (I) selon la voie de synthèse suivante.

Le composé de formule (II) peut être obtenu à partir du composé de formule (XI) : 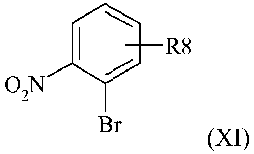 dans laquelle R8 peut être un atome d'halogène, un groupement alkyle ou halogénoalkyle.

On soumet le composé de formule (XI) à une réaction de Buchwald en présence de bis(2-diphénylphosphinophényl)éther et d'un catalyseur, comme défini ci-dessus, en milieu basique et apolaire en présence du composé (VII) tel que défini précédemment : 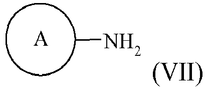 et on obtient le composé de formule (XII) suivante: 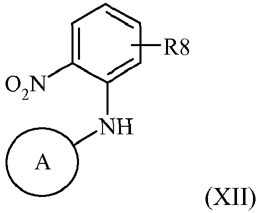 dans laquelle A et R8 sont tels que définis précédemment.

Le composé de formule (XII) est réduit par du chlorure d'étain en milieu polaire (Tet. Lett. 1984, 25 (8), 839) puis mis à réagir avec un dibromoalkane, par exemple du dibromopentane en milieu basique et apolaire (Bioorg. Med. Chem. Lett. 1996, 6 (5), 563) pour conduire au composé de formule (XIII) : 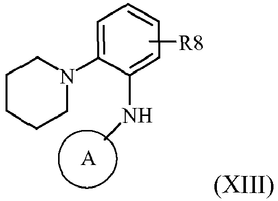 dans laquelle A et R8 sont tels que définis précédemment.

Le composé de formule (XIII) est mis en présence de nitrite de sodium en milieu acide puis réduit par un hydrure, par exemple l'hydrure de lithium et d'aluminium, pour donner le composé de formule (II) tel que défini précédemment :

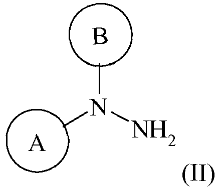

Les exemples suivants illustrent l'invention mais ne la limitent en aucune façon.

Les produits de départ utilisés sont des produits commerciaux ou des produits préparés selon des modes opératoires connus à partir de composés commerciaux ou connus par l'homme du métier. Les différentes préparations conduisent à des intermédiaires de synthèse utiles pour la préparation des composés de l'invention.

Les structures des composés décrits dans les exemples et dans les préparations ont été déterminées selon les techniques spectrophotométriques usuelles (résonance magnétique nucléaire (RMN), spectrométrie de masse (SM) dont électrospray (ES), ...) et la pureté a été déterminée par chromatographie liquide à haute performance (HPLC).

Abréviations utilisées dans les modes opératoires :
- CCM : chromatographie sur couche mince
- EDCI : chlorhydrate de 1-(3-diméthylaminopropyl)-3-éthylcarbodümide
- DMSO : diméthylsulfoxyde
- DMF : diméthylformamide
- NaCl : chlorure de sodium
- DIPEA : *N,N-*diisopropyléthylamine
- HOBt : 1-hydroxybenzotriazole
- TFA : acide trifluoroacétique
- THF : tétrahydrofurane
- DPEPhos : bis(2-diphénylphosphinophényl)éther
- NaOH : hydroxyde de sodium
- HCl : acide chlorhydrique
- Na₂CO₃ : carbonate de sodium
- Réactif de TEBBE : réactif d'oléfination de formule Cp₂Ti=CH₂
- Cp : cylcopentadiényle

### Préparation 1 : 4-Carboxyméthyl-2-méthoxy-benzoate de méthyle

Le 4-carboxyméthyl-2-méthoxy benzoate de méthyle peut être préparé selon la méthode décrite dans J. Med. Chem. 1998, 41, 5219 ou le brevet WO 0135900.

**Préparation 2 : 5-Bromo-4-carboxyméthyl-2-méthoxy-benzoate de méthyle** Le 5-bromo-4-carboxyméthyl-2-méthoxy benzoate de méthyle est obtenu à partir de la préparation 1 suivant le protocole décrit dans le brevet WO 0135900.

### Exemple 1 : 5-Bromo-2-méthoxy-4-[N'-(4-méthoxy-phényl)-N'-(2-méthyl-6-pipéridin-1-yl-phényl)-hydrazinocarbonylméthyl]-benzamide (1)

### Stade 1 : (4-Méthoxy-phényl)-(2-méthyl-6-nitro-phényl)-amine

A une solution de 50 g de 2-fluoro-3-nitrotoluène dans 1,1 L de DMSO sont ajoutés, à température ambiante, 59,5 g de para-anisidine (1,5 équivalents). Après 5 minutes d'agitation à température ambiante, 57,9 g de tert-butanoate de potassium (1,6 équivalents) sont additionnés au milieu réactionnel et l'ensemble est chauffé à 110 °C pendant 1 heure. Le milieu réactionnel est directement hydrolysé par ajout d'1 L de glace et d'eau puis la phase aqueuse est extraite plusieurs fois à l'acétate d'éthyle. Les phases organiques sont rassemblées, séchées sur sulfate de sodium, filtrées puis concentrées à sec. Le brut est chromatographié sur gel de silice (éther/cyclohexane : 0/100, 5/95 puis 20/80) pour fournir 34,95 g de composé attendu sous forme d'un solide rouge sombre.
Rendement : 42 %
RMN 1H (CDCl3, 400 MHz) δ (ppm) : 8,52 (s élargi, 1H), 7,98 (d, 1H), 7,36 (d, 1H), 6,97 (, 1H), 6,80 (s, 4H), 3,79 (d, 3H), 2,00 (s, 3H)

### Stade 2 : N²-(4-Méthoxy-phényl)-3-méthyl-benzène-1,2-diamine

A une solution de 72,0 g du composé précédemment obtenu dans 900 mL d'éthanol sont ajoutés sous argon 314,5 g de chlorure d'étain (5 équivalents). Le milieu réactionnel est chauffé à reflux pendant 2 heures. Le milieu est ensuite refroidi à température ambiante puis concentré sous pression réduite. L'ensemble est repris dans 600 mL d'acétate d'éthyle et 300 mL d'eau. Le milieu est ensuite basifié jusqu'à pH 8 à l'aide d'une solution de NaOH 50%. Les deux phases sont séparées et la phase aqueuse est extraite plusieurs fois à l'acétate d'éthyle. Les phases organiques sont réunies, séchées sur sulfate de sodium, filtrées et évaporées à sec. 52,27 g du composé attendu sont obtenus sous forme d'un solide brun. Le produit est engagé sans purification supplémentaire dans l'étape suivante.
Rendement : 90 %
RMN 1H (CDCl3, 400 MHz) δ (ppm) : 7,01 (t, 1H), 6,77 (d, 2H), 6,67 (m, 2H), 6,56 (d, 2H), 3,76 (s, 3H), 2,18 (s, 3H)

### Stade 3 : (4-Méthoxy-phényl)-(2-méthyl-6-pipéridin-1-yl-phényl)-amine

A une solution de 50,0 g du composé obtenu précédemment, dans 800 mL de toluène anhydre sont ajoutés successivement 92,0 mL de DIPEA (2,4 équivalents), 29,85 mL de 1,5-dibromopentane (1,0 équivalent) et 5 g d'iodure de sodium (environ 10% massique). Le milieu est agité à reflux, sous argon, pendant 12 heures. Le brut réactionnel est dilué dans 500 mL d'eau et la phase aqueuse résultante est extraite plusieurs fois à l'acétate d'éthyle. Les phases organiques sont rassemblées, séchées sur sulfate de sodium, filtrées et concentrées à sec. Le brut est chromatographié sur gel de silice (acétate d'éthyle/cyclohexane : 2/98) pour fournir 22,75 g de composé attendu sous forme d'un solide brun.
Rendement : 36 %
RMN 1H (CDCl3, 400 MHz) δ (ppm) : 6,97 (m, 3H), 6,77 (d, 2H), 6,70 (d, 2H), 6,16 (s élargi, 1H), 3,77 (s, 3H), 2,74 (m, 4H), 2,09 (s, 3H), 1,61 (m, 4H), 1,53 (m, 2H)

### Stade 4 : N-Nitroso-(4-méthoxy-phényl)-N-(2-méthyl-6-pipéridin-1-yl-phényl)-amine

A une solution thermostatée à 10°C de 20,6 g du composé précédent dans 250 mL d'acide acétique est ajoutée goutte à goutte une solution de 27,81 g de nitrite de sodium (5,8 équivalents) dans 250 mL d'eau. Le milieu est laissé sous agitation à température ambiante, sous argon, pendant 25 minutes. Le brut est dilué dans 200 mL d'eau puis la solution aqueuse résultante est versée sur 100 g environ de carbonate de sodium solide. La phase aqueuse est extraite plusieurs fois au dichlorométhane et les phases organiques sont rassemblées, séchées sur sulfate de sodium, puis évaporées à sec. 20,0 g de brut sont obtenus puis engagés dans l'étape suivante sans purification supplémentaire.
Rendement : 87 %
RMN 1H (CDCl3, 400 MHz) δ (ppm) : 7,29 (m, 3H), 6,99 (d, 2H), 6,90 (d, 2H), 3,84 (s, 3H), 2,67 (m, 2H), 2,52 (m, 2H), 1,95 (s, 3H), 1,36 (m, 4H), 1,25 (m, 2H)

### Stade 5 : N-(4-Méthoxy-phényl)-N-(2-méthyl-6-pipéridin-1-yl-phényl)-hydrazine

A une solution de 7,0 g du composé précédent dans 85 mL d'éther anhydre est ajouté lentement sous argon une solution 1 N de 86,05 mL d'hydrure de lithium et d'aluminium dans l'éther (4 équivalents). Le milieu réactionnel est agité à reflux pendant 1 heure au bout de laquelle la réaction est terminée (suivi CCM). Le brut réactionnel est neutralisé par ajout goutte à goutte de 100 mL d'acétate d'éthyle puis 200 mL d'eau. La phase aqueuse est extraite plusieurs fois à l'acétate d'éthyle et les phases organiques sont rassemblées, séchées sur sulfate de sodium, filtrées et concentrées à sec. Le brut est chromatographié sur gel de silice (acétate d'éthyle/cyclohexane : 1/99) pour fournir 4,61 g de composé attendu sous forme d'une poudre rouge-orange.
Rendement : 69 %
RMN 1H (CDCl3, 400 MHz) δ (ppm) : 7,14 (t, 1H), 6,99 (dd, 2H), 6,77 (s, 4H), 4,90 (s élargi, 2H), 3,76 (s, 3H), 2,80 (m, 4H), 2,09 (s, 3H), 1,57 (m, 6H)

### Stade 6 : 5-Bromo-2-méthoxy-4-[N'-(4-méthoxy-phényl)-N'-(2-méthyl-6-pipéridin-1-yl-phényl)-hydrazinocarbonylméthyl]-benzoate de méthyle

A une solution de 5,28 g de composé obtenu précédemment dans 60 mL de DMF sont ajoutés successivement, 5,65 g de composé obtenu à la préparation 2 (1,1 équivalents), 2,52 g d'HOBt (1,1 équivalents), 3,57 g d'EDCI (1,1 équivalents) et 2,35 mL de triéthylamine (1,0 équivalent). Après 15 minutes d'agitation à 100 °C, la réaction est terminée (suivi CCM). Le milieu réactionnel est refroidi puis versé sur de la glace. Le précipité formé est filtré puis repris dans le dichlorométhane. La phase organique résultante est séchée sur sulfate de sodium, filtrée et évaporée à sec. Le brut est chromatographié sur gel de silice (acétate d'éthyle/cyclohexane : 1/9) pour fournir 8,81 g de composé attendu sous forme d'une poudre brune.
Rendement : 87 %
RMN ¹H (CDCl3, 400 MHz) δ (ppm) : 10,06 et 9,92 (2s, 1H), 8,02 et 7,98 (2s, 1H), 7,19 (m, 1H), 7,02 (m, 2H), 6,91 (s, 1H), 6,75 (m, 2H), 6,54 (d, 2H), 3,89 (s, 3H), 3,87 et 3,78 (m, 2H), 3,74 (s, 6H), 3,71 (m, 2H), 2,79 (m, 2H), 2,40 (s, 3H), 1,45 (m, 6H)

### Stade 7: Chlorhydrate d'acide 5-bromo-2-méthoxy-4-[N'-(4-méthoxy-phényl)-N'-(2-méthyl-6-pipéridin-1-yl-phényl)-hydrazinocarbonylméthyl]-benzoïque

A une solution de 6,99 g du composé précédemment obtenu dans 160 mL de THF est ajoutée une solution de 850 mg d'hydroxyde de lithium (3 équivalents) dans 50 mL d'eau. Le milieu est laissé sous agitation à température ambiante et sous argon pendant 12 heures. Le THF est alors évaporé sous pression réduite et une solution d'acide chlorhydrique 1 M est ajoutée goutte à goutte. Le précipité formé est filtré, lavé par une solution d'acide chlorhydrique 1 M puis repris dans le dichlorométhane. Après évaporation du solvant sous pression réduite, le solide jaune pâle est purifié par chromatographie sur gel de silice (dichlorométhane/méthanol : 98/2 à 95/5). La poudre blanche résultante est ensuite solubilisée dans un minimum de mélange THF/eau (1:4) puis re-précipitée par ajout goutte à goutte d'une solution d'acide chlorhydrique 1 M. Le précipité formé est filtré, lavé avec une solution d'acide chlorhydrique 1 M et séché sous vide à 35 °C pendant 48 heures. 4,35 g du composé attendu sont obtenus sous forme d'un solide blanc.
Rendement : 60 %
HPLC : 98,6 %
SM : MH⁺ 582/584
RMN ¹H (DMSO+TFA, 400 MHz) δ (ppm) : 7,90 (d, 1H), 7,79 (s, 1H), 7,68 (t, 1H), 7,61 (d, 1H), 7,26 (s, 1H), 6,88 (d, 2H), 6,62 (d, 2H), 4,14 (d, 1H), 3,95 (m, 2H), 3,76 (s, 3H), 3,69 (s, 3H), 3,42 (m, 1H), 3,30 (d, 1H), 3,16 (d, 1H), 2,21 (s, 3H), 1,85 (m, 4H), 1,48 (m, 2H)

### Stade 8: 5-Bromo-2-méthoxy-4-[N'-(4-méthoxy-phényl)-N'-(2-méthyl-6-pipéridin-1-yl-phényl)-hydrazinocarbonylméthyl]-benzoate de 2,5-dioxo-pyrrolidin-1-yle

A une solution de 117 mg du composé précédemment obtenu dans 570 µL de dichlorométhane, sont additionnés consécutivement à température ambiante 80 µL de triéthylamine (3,0 équivalents), 32 mg de *N*-hydroxysuccinimide (1,5 équivalents) puis 54 mg d'EDCI (1,5 équivalents). Après 18 heures d'agitation, le milieu réactionnel est dilué dans 10 mL de chloroforme. La phase organique résultante est lavée plusieurs fois à l'eau puis par une solution saturée de NaCl, séchée sur sulfate de sodium, évaporée à sec. Le brut est chromatographié sur gel de silice (dichlorométhane/méthanol : 98/2) pour fournir 76 mg du composé attendu sous forme d'un solide blanc qui est engagé dans l'étape suivante sans purification supplémentaire.
Rendement : 59 %

### Stade 9: 5-Bromo-2-méthoxy-4-[N'-(4-méthoxy-phényl)-N'-(2-méthyl-6-pipéridin-1-yl-phényl)-hydrazinocarbonylméthyl]-benzamide (1)

50 mg d'une solution du composé obtenu précédemment dans 500 µL de dioxane est purgée sous argon. Un courant d'ammoniac gazeux est maintenu pendant 30 minutes à température ambiante puis le milieu réactionnel est agité pendant 2 heures puis évaporé à sec. Le solide blanc obtenu est repris dans 10 mL d'acétate d'éthyle. La phase organique résultante est lavée plusieurs fois à l'eau, avec une solution saturée de NaCl, séchée sur sulfate de sodium, évaporée à sec. Le brut est chromatographié sur gel de silice (dichlorométhane/méthanol : 98/2) pour fournir 36 mg du composé attendu sous forme d'un solide blanc.
Rendement : 83 %
HPLC : 96,3 %
SM : MH⁺ 581/583
RMN ¹H (DMSO+TFA, 200 MHz) δ (ppm) : 7,95 (s, 1H), 7,91 (s, 1H), 7,67 (m, 3H), 7,26 (s, 1H), 6,91 (d, 2H), 6,61 (d, 2H), 3,75-4,16 (m + dd, 3H), 3,84 (s, 3H), 3,70 (s, 3H), 3,10-3,45 (m, 3H), 2,20 (s, 3H), 1,45-1,92 (m, 6H)

### Exemple 2 : 5-Bromo-2-méthoxy-4-[N'-(4-méthoxy-phényl)-N'-(2-méthyl-6-pipéridin-1-yl-phényl)-hydrazinocarbonylméthyl]-N,N-diméthyl-benzamide (2)

A une solution de 85 mg du composé obtenu au stade 8 de l'exemple 1 dans 500 µL de THF sont additionnés à température ambiante 2 mL (29 équivalents) d'une solution de diméthylamine 2 M dans le THF. Le milieu réactionnel est ensuite agité pendant 2 heures avant d'être évaporé à sec. Le solide blanc obtenu est repris dans 10 mL d'acétate d'éthyle. La phase organique résultante est lavée plusieurs fois à l'eau puis par une solution saturée de NaCl, séchée sur sulfate de sodium, évaporée à sec. Le brut est chromatographié sur gel de silice (dichlorométhane/méthanol : 98/2) pour fournir 45 mg du composé attendu sous forme d'un solide blanc.
Rendement : 53 %
HPLC : 97 %
SM : MH⁺ 609/611
RMN ¹H (DMSO+TFA, 200 MHz) δ (ppm) : 7,91 (d, 1H), 7,70 (m, 2H), 7,38 (s, 1H), 7,19 (s, 1H), 6,88 (d, 2H), 6,81 (d, 2H), 4,09 (d, 1H), 3,93 (m, 1H), 3,88 (d, 1H), 3,75 (s, 3H), 3,70 (s, 3H), 3,10-3,50 (m, 3H), 2,95 (s, 3H), 2,75 (s, 3H), 2,20 (s, 3H), 1,65-1,95 (m, 4H), 1,49 (m, 2H)

### Exemple 3 : [2-Bromo-5-méthoxy-4-(4-méthyl-pipérazine-1-carbonyl)-phényl]-acétic acid N'-(4-méthoxy-phényl)-N'-(2-méthyl-6-pipéridin-1-yl-phenyl)-hydrazide (3)

A 92 mg d'une solution du composé obtenu au stade 8 de l'exemple 1 dans 450 µL de dichlorométhane sont additionnés à température ambiante 82 µL de *N*-méthylpipérazine (5 équivalents). Le milieu réactionnel est agité pendant 2 heures avant d'être évaporé à sec. Le solide blanc obtenu est repris dans 10 mL d'acétate d'éthyle. La phase organique résultante est lavée plusieurs fois à l'eau puis par une solution saturée de NaCl, séchée sur sulfate de sodium, évaporée à sec. Le brut est chromatographié sur gel de silice (dichlorométhane/méthanol : 98/2) pour fournir 53 mg du composé attendu sous forme d'un solide blanc.
Rendement : 54 %
HPLC : 98,1 %
SM : MH⁺ 664/666
RMN ¹H (DMSO+TFA, 200 MHz) δ (ppm) : 9,55 (s, 1H), 7,38 (s, 1H), 6,99-7,20 (m, 4H), 6,79 (d, 2H), 6,50 (d, 2H), 3,54-3,80 (m, 2s, m, 11H), 3,10 (m, 2H), 2,70 (m, 1H), 2,30 (m, 4H), 2,22 (s, 3H), 2,16 (s, 3H), 1,40 (m, 4H), 1,25 (m, 2H)

### Exemple 4: 4-[N'-(4-Acétyl-phényl)-N'-(2-méthyl-6-pipéridin-1-yl-phényl)-hydrazinocarbonyl méthyl]-5-bromo-2-méthoxy-benzamide (4)

### Stade 1 : 1-[4-(2-Méthyl-6-nitro-phénylamino)-phényl]-éthanone

Dans un ballon sous atmosphère d'argon sont placés consécutivement 2,16 g de 2-bromo-3-nitrotoluène, 1,69 g de 4-aminoacétophénone (1,25 équivalents), 229 mg de dipalladium trisdibenzylidèneacétone (0,025 équivalents), 269 mg de DPEPhos (0,05 équivalents) et 4,07 g de carbonate de césium (1,25 équivalents). Le toluène est additionné à température ambiante. Le milieu réactionnel hétérogène est purgé à l'argon puis porté à reflux pendant 16 heures. Après retour à température ambiante, de l'eau et de l'acétate d'éthyle sont additionnés. Après séparation des phases, la phase organique est lavée à l'eau puis avec une solution saturée de NaCl, séchée sur sulfate de sodium, filtrée puis évaporée à sec. L'huile rouge obtenue est purifiée par chromatographie sur silice (acétate d'éthyle/cyclohexane : 5/95 à 10/90) pour fournir 2,29 g du composé attendu sous forme d'une huile rouge.
Rendement : 85 %
RMN 1H (CDCl3, 200 MHz) δ (ppm) : 7,95 (m, 4H), 7,51 (d, 1H), 7,19 (d, 1H), 6,68 (d, 2H), 2,53 (s, 3H), 2,17 (s, 3H)

### Stade 2 : 1-[4-(2-Amino-6-méthyl-phénylamino)-phényl]-éthanone

Le produit (2,02 g) est obtenu selon le procédé du stade 2 de l'exemple 1, en utilisant 2,29 g du dérivé précédent comme produit de départ et 9,56 g de chlorure d'étain dans 30 mL d'éthanol.
Rendement : 99 %
RMN 1H (CDCl3, 200 MHz) δ (ppm) : 7,81 (d, 2H), 7,06 (t, 1H), 6,68 (d, 2H), 6,55 (d, 2H), 5,42 (s élargi, 1H), 2,50 (s, 3H), 2,15 (s, 3H)

### Stade 3 : 1-[4-(2-Méthyl-6-pipéridin-1-yl-phénylamino)-phényl]-éthanone

Le produit (1,36 g) est obtenu selon le procédé du stade 3 de l'exemple 1, en utilisant 2,02 g du dérivé précédent comme produit de départ, 3,5 mL de DIPEA, 1,20 mL de 1,5-dibromopentane et 200 mg d'iodure de sodium.
Rendement : 52 %
RMN 1H (CDCl3, 200 MHz) δ (ppm) : 7,84 (d, 2H), 7,08 (d, 1H), 6,97 (m, 2H), 6,64 (d, 2H), 6,35 (s élargi, 1H), 2,72 (m, 4H), 2,52 (s, 3H), 2,14 (s, 3H), 1,55 (m, 6H)

### Stade 4 : [4-(2-Méthyl-[1,3]dithian-2-yl)-phényl]-(2-méthyl-6-pipéridin-1-yl-phényl)-amine

A une solution de 1,36 g de composé obtenu précédemment dans 22 mL de dichlorométhane sont additionnés successivement à température ambiante 550 µL de 1,3-propanedithiol (1,25 équivalents) puis 920 µL d'étherate de trifluorure de bore (1,5 équivalents). Après 18 heures d'agitation à température ambiante, la réaction est stoppée par ajout de 50 mL d'une solution de NaOH 2 M. Après séparation des phases, la phase aqueuse est extraite plusieurs fois au dichlorométhane. Les phases organiques sont réunies, lavées avec une solution saturée de NaCl, séchées sur sulfate de sodium, filtrées puis évaporées à sec pour fournir 1,55 g du composé attendu sous forme d'une mousse blanche.
Rendement : 89 %
RMN 1H (CDCl3, 200 MHz) δ (ppm) : 7,67 (d, 2H), 6,99 (m, 3H), 6,67 (d, 2H), 2,77 (m, 8H), 2,14 (s, 3H), 1,95 (m, 2H), 1,87 (s, 3H), 1,66 (m, 4H), 1,53 (m, 2H)

### Stade 5 : N-Nitroso-[4-(2-méthyl-[1,3]dithian-2-yl)-phényl]-(2-méthyl-6-pipéridin-1-yl-phényl)-amine

A une solution de 856 mg du composé précédemment obtenu dans 6 mL d'acide acétique est ajoutée goutte à goutte une solution de 1,17 g de nitrite de sodium (5,8 équivalents) dans 6 mL d'eau. Il se forme un précipité qui est dissout par addition successive de 6 mL de dichlorométhane et 6 mL de méthanol. Le milieu réactionnel est laissé sous agitation à température ambiante, sous argon, pendant 2 heures puis versé sur 10 g de carbonate de sodium solide. Le brut est dilué par 40 mL d'eau et 40 mL de dichlorométhane. Les phases sont séparées puis la phase aqueuse est extraite plusieurs fois au dichlorométhane. Les phases organiques sont rassemblées, séchées sur sulfate de sodium, puis évaporées à sec pour fournir 839 mg du composé désiré sous forme d'une mousse rouge pâle. Ce dérivé nitroso est engagé dans l'étape suivante sans purification supplémentaire.
Rendement : 91 %
RMN 1H (CDCl3, 200 MHz) δ (ppm) : 7,93 (d, 2H), 7,33 (m, 3H), 7,00 (t, 2H), 2,49-2,77 (m, 8H), 1,99 (m, 2H), 1,96 (s , 3H), 1,81 (s, 3H), 1,33 (m, 4H), 1,19 (m, 2H)

### Stade 6 : N-[4-(2-Méthyl-[1,3]dithian-2-yl)-phényl]-N-(2-méthyl-6-pipéridin-1-yl-phényl)-hydrazine

Le produit (333 mg) est obtenu selon le procédé du stade 5 de l'exemple 1, en utilisant 836 mg du dérivé précédent comme produit de départ et 8 mL d'une solution 1 M d'hydrure de lithium et d'aluminium dans l'éther diéthylique.
Rendement : 41 %
RMN 1H (CDCl3, 200 MHz) δ (ppm) : 7,64 (d, 2H), 7,18 (m, 1H), 6,99 (t, 2H), 6,79 (d, 2H), 2,78 (m, 8H), 2,10 (s, 3H), 1,93 (m, 2H), 1,86 (s, 3H), 1,56 (m, 6H)

### Stade 7: 5-Bromo-2-méthoxy-4-[N'-[4-(2-méthyl-[1,3]dithian-2-yl)-phényl]-N'-(2-méthyl-6-pipéridin-1-yl-phényl)-hydrazinocarbonylméthyl]-benzoate de méthyle

Le produit (320 mg) est obtenu selon le procédé du stade 6 de l'exemple 1, en utilisant 333 mg de l'hydrazine précédente et 267 mg de l'acide de la préparation 2 en présence de 119 mg d'HOBt, 169 mg d'EDCI et 130 µL de triéthylamine dans 2,4 mL de DMF.
Rendement : 57 %
RMN ¹H (CDCl3, 200 MHz) δ (ppm) : 9,76 (s, 1H), 8,00 (s, 1H), 7,67 (d, 2H), 6,92-7,21 (m, 4H), 6,57 (d, 2H), 3,91 (m, 2H), 3,96 (s, 3H), 3,73 (s, 3H), 2,71 (m, 6H), 2,43 (s, 3H), 2,36 (m, 2H), 1,93 (m, 2H), 1,78 (s, 3H), 1,22-1,44 (m, 6H)

### Stade 8 : 4-[N'-( 4-Acétyl-phényl)-N'-(2-méthyl-6-pipéridin-1-yl-phényl)-hydrazinocarbonylméthyl]-5-bromo-2-méthoxy-benzoate de méthyle

A une solution de 320 mg du composé précédemment obtenu dans 5 mL d'un mélange THF/eau (9 : 1) sont additionnés consécutivement à température ambiante 204 mg d'oxyde de mercure (II) (2,0 équivalents) puis 130 µL d'étherate de trifluorure de bore (2,0 équivalents). L'agitation est poursuivie pendant 16 heures puis la réaction est stoppée par ajout de 20 mL d'une solution de soude 2 M. La phase aqueuse résultante est extraite plusieurs fois à l'acétate d'éthyle. Les phases organiques sont réunies, lavées à l'eau et avec une solution saturée de NaCl, séchées sur sulfate de sodium, filtrées puis évaporés à sec. Le brut est chromatographié sur gel de silice (chloroforme 100%) pour fournir 210 mg du composé attendu sous forme d'un solide blanc.
Rendement : 73 %
RMN ¹H (CDCl3, 200 MHz) δ (ppm) : 9,78 (s, 1H), 7,98 (s, 1H), 7,82 (d, 2H), 7,21 (d, 1H), 7,01 (t, 2H), 6,88 (s, 1H), 6,60 (d, 2H), 3,85 (s, 3H), 3,75 (m, 2H), 3,73 (s, 3H), 2,72 (m, 2H), 2,36-2,49 (s+m+s, 8H), 1,39 (m, 6H)

### Stade 9 : Acide [N'-(4-acétyl-phényl)-N'-(2-méthyl-6-pipéridin-1-yl-phényl)-hydrazine carbonylméthyl]-5-bromo-2-méthoxy-benzoïque

A une solution de 210 mg du composé précédemment obtenu dans 3,5 mL de THF sont additionnés à température ambiante 41 mg d'hydroxyde de lithium (5 équivalents) en solution dans 1,4 mL d'eau. Un solide blanc précipite dans le milieu réactionnel qui est solubilisé par addition de 500 µL de méthanol. L'agitation est poursuivie 18 heures puis le milieu réactionnel est dilué dans 50 mL d'acétate d'éthyle. La phase organique résultante est lavée à l'eau puis avec une solution saturée de NaCl, séchée sur sulfate de sodium, évaporée à sec. Le brut est chromatographié sur gel de silice (dichlorométhane/méthanol : 96/4) pour fournir 142 mg du composé attendu sous forme d'une mousse blanche.
Rendement : 69 %
HPLC : 96,3 %
SM : MH⁺ 594/596
RMN ¹H (CDCl3, 200 MHz) δ (ppm) : 9,98 (s, 1H), 8,36 (s, 1H), 7,82 (d, 2H), 7,25 (s, 1H), 7,03 (m, 3H), 6,60 (d, 2H), 3,94 (s, 3H), 3,78 (s, 2H), 2,80 (m, 2H), 2,37-2,51 (s+m+s, 8H), 1,57 (m, 6H)

### Stade 10 : 4-[N'-(4-Acétyl-phényl)-N'-(2-méthyl-6-pipéridin-1-yl-phényl)-hydrazino carbonylméthyl]-5-bromo-2-méthoxy-benzamide (4)

A une solution de 75 mg du composé précédent dans 200 µL de THF sont ajoutés successivement à température ambiante 20 mg d'HOBt, 28 mg d'EDCI (1,1 équivalents) puis 300 µL (4,8 équivalents) d'ammoniaque 2 M en solution dans le THF. Le milieu réactionnel est agité à température ambiante pendant 18 heures puis dilué à l'acétate d'éthyle. La phase organique résultante est lavée plusieurs fois à l'eau puis par une solution saturée de NaCl, séchée sur sulfate de sodium, filtrée et évaporée à sec. Le brut est chromatographié sur gel de silice (dichlorométhane/méthanol : 98/2) pour fournir 70 mg du composé attendu sous forme d'un solide blanchâtre. Une fraction de la mousse blanche obtenue est solubilisée dans 1 mL d'éther diéthylique. Après ajout de 100 µL d'une solution d'acide chlorhydrique 4 M dans le dioxane, puis filtration, le chlorhydrate du composé attendu est obtenu sous forme d'un solide blanc.
Rendement (forme neutre) : 93 %
HPLC : 96,75 %
SM : MH⁺ 593/595
RMN ¹H - forme chlorhydrate - (DMSO, 200 MHz) δ (ppm) : 7,92 (m, 4H), 7,69 (m, 4H), 7,36 (s, 1H), 6,74 (s élargi, 2H), 4,04-4,28 (dd+m, 3H), 3,86 (s, 3H), 3,16-3,56 (m, 3H), 2,24 (s, 3H), 1,77 (m, 4H), 1,46 (m, 2H).

### Exemple 5 : (4-Acétyl-2-bromo-5-méthoxy-phényl)-acétic acid N'-(4-méthoxy-phényl)-N'-(2-méthyl-6-pipéridin-1-yl-phényl)-hydrazide (5)

### Stade 1 : [2-Bromo-5-méthoxy-4-(1-méthoxy-vinyl)-phényl]-acétic acid N'-(4-méthoxy-phényl)-N'-(2-méthyl-6-pipéridin-1-yl-phényl)-hydrazide

A une solution de 200 mg du composé obtenu au stade 6 de l'exemple 1 dans 2 mL de THF sont ajoutés, à température ambiante, 177 µL d'une solution du réactif de TEBBE à 4 M dans le toluène. Après 20 heures d'agitation à température ambiante, le milieu réactionnel est basifié à l'aide de 15 mL d'une solution saturée de Na₂CO₃ puis la phase aqueuse est extraite à l'acétate d'éthyle (3 x 20 mL). Les phases organiques sont rassemblées, séchées sur sulfate de sodium, filtrées puis concentrées à sec. Le brut est chromatographié sur gel de silice (cyclohexane/acétate d'éthyle : 90/10) pour fournir 138 mg de composé attendu pur sous forme d'un solide blanc.
Rendement : 68 %
SM : MH⁺ 593/595

### Stade 2 : (4-Acétyl-2-bromo-5-méthoxy-phényl)-acétic acid N'-(4-méthoxy-phényl)-N'-(2-méthyl-6-pipéridin-1-yl-phényl)-hydrazide (5)

A une solution de 138 mg du composé précédemment obtenu dans 2 mL de THF est ajouté 1 mL d'une solution d'HCl 1N. Après 4 heures d'agitation à température ambiante, le milieu réactionnel est basifié à l'aide de 20 mL d'une solution saturée de Na₂CO₃ puis la phase aqueuse est extraite à l'acétate d'éthyle (3 x 20 mL). Les phases organiques sont rassemblées, séchées sur sulfate de sodium, filtrées puis concentrées à sec. Le brut est chromatographié sur gel de silice (cyclohexane/acétate d'éthyle : 80/20) pour fournir 96 mg du composé attendu sous forme d'un solide beige.
Rendement : 74 %
HPLC : 96,26 %
SM : MH⁺ 579/581
RMN ¹H (CD₃OD, 200 MHz) δ (ppm) : 7,80 (s, 1H), 7,00-7,3 (m, 4H), 6,65 (dd, 4H), 3,83 (s+m, 8H), 3,72 (s, 3H), 2,75 (m, 2H), 2,56 (s, 3H), 2,35 (s+m, 5H), 1,15-1,45 (m, 6H)

### Exemple 6 : 5-Bromo-2-N-diméthoxy-4-[N-(4-méthoxy-phényl)-N'-(2-méthyl-6-pipéridin-1-yl-phényl)-hydrazinocarbonylméthyl]-N-méthyl-benzamide (9)

A une suspension de 200 mg du composé obtenu au stade 7 de l'exemple 1 sous forme neutre dans 3 mL de dichlorométhane anhydre sont ajoutés, goutte à goutte à 0°C, 30 µL de chlorure de méthanesulfonyle. Le milieu devient alors homogène. Après 20 minutes d'agitation à 0°C, 38 mg de chlorhydrate de la N,O-diméthylhydroxylamine sont ajoutés au milieu réactionnel et l'ensemble est placé sous agitation à 0°C pendant 30 minutes supplémentaires au bout desquelles la réaction n'est pas terminée (selon le suivi CCM). Une quantité supplémentaire de 38 mg de chlorhydrate de la N,O-diméthylhydroxylamine sont alors ajoutés et le milieu réactionnel est placé sous agitation à température ambiante pendant une heure. Le milieu devient hétérogène et se présente sous forme d'une suspension blanche. Le brut réactionnel est ensuite hydrolysé avec 10 mL d'eau puis la phase aqueuse résultante est extraite au dichlorométhane (3 x 20 mL). Les phases organiques sont rassemblées, séchées sur sulfate de sodium, filtrées et concentrées à sec. Le brut est chromatographié sur gel de silice (cyclohexane/acétate d'éthyle : 90/10) pour fournir 60 mg de composé attendu sous forme d'un solide beige.
Rendement : 29 %
SM : MH⁺ 624/626
RMN ¹H (DMSO + TFA, 200 MHz) δ (ppm) : 7,60-7,95 (m, 3H), 7,47 (s, 1H), 7,20 (s, 1H), 6,75 (dd, 4H), 3,80-4,15 (m, 3H), 3,74 (s, 3H), 3,71 (s, 3H), 3,10-3,55 (m, 9H), 2,20 (s, 3H), 1,40-1,95 (m, 6H)

### Exemple 7 : 5-Bromo-N-tert-butyl-2-méthoxy-4-[N'-(4-méthoxy-phényl)-N'-(2-méthyl-6-pipéridin-1-yl-phényl)-hydrazinocarbonylméthyl]-benzamide (10)

A une suspension de 300 mg du composé obtenu au stade 7 de l'exemple 1 sous forme neutre dans 4 mL de dichlorométhane anhydre sont ajoutés successivement 120 µL de chlorure d'oxalyle 3 gouttes de DMF. Le milieu devenu homogène est agité, à température ambiante, sous argon, pendant 1 heure avant d'être évaporé à sec puis placé à nouveau sous argon. Le brut réactionnel est alors dilué dans 4 mL de dichlorométhane avant d'additionner goutte à goutte 72 µL de *tert*-butylamine. Après 1 heure d'agitation à température ambiante, le milieu réactionnel est basifié à l'aide de 20 mL d'une solution saturée de Na₂CO₃ puis la phase aqueuse est extraite à l'acétate d'éthyle (3 x 30 mL). Les phases organiques sont rassemblées, séchées sur sulfate de sodium, filtrées et concentrées à sec. Le brut est chromatographié sur gel de silice (cyclohexane/acétate d'éthyle : 80/20) pour fournir 95 mg de composé attendu sous forme d'un solide beige.
Rendement : 29 %
SM : MH⁺ 636/638
RMN ¹H (DMSO + TFA, 200 MHz) δ (ppm) : 7,60-7,95 (m, 6H), 7,26 (s, 1H), 6,75 (dd, 4H), 3,95-4,10 (m, 6H), 3,85 (s, 3H), 3,70 (s, 3H), 3,10-3,50 (m, 3H), 2,21 (s, 3H), 1,40-1,95 (m, 6H), 1,35 (s, 9H)

### Exemple 8 : (2-Bromo-4-cyano-5-méthoxy-phényl)-acétic acid N'-(4-méthoxy-phényl)-N'-(2-méthyl-6-pipéridin-1-yl-phényl)-hydrazide (11)

A une solution de 490 mg du composé obtenu au stade 9 de l'exemple 1 dans 4 mL de dichlorométhane anhydre sont ajoutés successivement à -78°C, 96 µL de DMSO et 128 mg de chlorure d'oxalyle. Après 15 minutes d'agitation à -78°C, 350 µL de triéthylamine sont additionnés lentement au milieu réactionnel à -78°C. Après 30 minutes d'agitation supplémentaires à -78°C, le milieu réactionnel est hydrolysé avec 10 mL d'eau puis la phase aqueuse est extraite au dichlorométhane (3 x 15 mL). La phase organique résultante est séchée sur sulfate de sodium, filtrée et évaporée à sec. Le brut est chromatographié sur gel de silice (cyclohexane/acétate d'éthyle :80/20) pour fournir 356 mg de composé attendu sous forme d'une poudre blanche.
Rendement : 75 %
SM : MH⁺ 562/564
RMN ¹H (DMSO + TFA, 200 MHz) δ (ppm) : 8,08 (s, 1H), 7,60-7,95 (m, 3H), 7,40 (s, 3H), 6,75 (dd, 4H), 3,95-4,20 (m, 3H), 3,88 (s, 3H), 3,70 (s, 3H), 3,10-3,50 (m, 3H), 2,20 (s, 3H), 1,40-1,90 (m, 6H)

### RESULTATS D'ACTIVITE BIOLOGIQUE

L'activité des molécules contre le virus du papillome peut être évaluée dans différents tests *in vitro* et cellulaires comme ceux décrits par Chiang et al. (1992), Proc. Natl. Acad. Sci. USA, 89 :5799-5803 ou encore par White et al. (2003), Journal of Biological Chemistry, 278 :26765-26772.

### Exemple 9 : études pharmacologiques des composés de l'invention dans des tests cellulaires d'interaction entre les protéines virales E1 et E2 et de réplication de l'ADN viral des VPH

Une première série de tests évalue l'interaction entre les protéines E1 et E2 des VPH dans les cellules humaines. Une deuxième série de tests mesure la réplication de l'ADN génomique viral dans des cellules humaines.

Les tests d'interaction entre E1 et E2 s'apparentent aux tests souvent appelés 'mammalian 2 Hybrid'. Ils reposent sur la co-transfection d'un vecteur rapporteur contenant des sites de fixation à l'ADN pour la protéine E2 dans le promoteur contrôlant l'expression du gène rapporteur, et de vecteurs d'expression codant pour les protéines E1 et E2 de VPH, les protéine E1 étant fusionnées au domaine transactivateur VP16. Ces tests permettent de suivre l'interaction entre les protéines E1 et E2, cette interaction étant une étape nécessaire à la réplication du génome des VPH.

Les tests de réplication de l'ADN génomique viral reposent sur la co-transfection d'un vecteur rapporteur contenant une origine de réplication virale (ori) et de vecteurs d'expression codant pour les protéines E1 et E2 de VPH. Ils permettent de suivre l'ensemble des fonctions biologiques de E1 et E2 nécessaires à la réplication du génome des VPH.

Pour les tests d'interaction entre E1 et E2, il a été construit un vecteur rapporteur contenant plusieurs sites de fixation à l'ADN pour la protéine E2 (le palindrome 5' ACCGNNNNCGGT - 3') en amont du promoteur minimal MLP (Adenovirus Major Late Promoter) contrôlant la transcription du gène codant pour la luciférase de luciole. Il a aussi été construit des vecteurs d'expression des protéines E1 des VPH fusionnées en N-terminal avec le domaine transactivateur VP16 du virus HSV-1. La co-transfection de ce vecteur rapporteur contenant des sites E2 et de vecteurs d'expression des protéines E2 des VPH conduit à une augmentation marginale de l'activité luciférase. La co-transfection de ce vecteur rapporteur contenant des sites E2, de vecteurs d'expression des protéines E2 des VPH et de vecteurs d'expression des protéines E1 fusionnées au domaine VP16 permet la formation dans les cellules du complexe protéique E2 / E1-VP16 fortement transactivateur, et conduit à une forte augmentation de l'activité luciférase. Ceci traduit l'interaction entre les protéines E1 et E2 dans les cellules.

Pour les tests de réplication de l'ADN génomique viral, il a été construit un vecteur rapporteur 'réplicon' contenant l'origine de réplication virale de VPH11 / VPH6 (appelée aussi LCR qui portent des sites de fixation des protéines E1 et E2 du VPH) et le gène codant pour la luciférase de luciole sous le contrôle transcriptionnel du promoteur de SV40. Il a été vérifié que la présence de l'origine de réplication du VPH n'a aucun effet transcriptionnel sur l'expression du gène de la luciférase, ceci en présence ou en absence des protéines virales E1 ou E2. La co-transfection de ce vecteur-réplicon et de vecteurs d'expression des protéines E1 et E2 de VPH conduit à une augmentation de l'activité luciférase dépendante de la présence de E1 et de E2 et traduit l'augmentation du nombre de vecteurs rapporteurs. Ceci est dû à l'activité des protéines virales E1 et E2 qui permettent la réplication, dans les cellules mammifères, de ce vecteur-réplicon contenant une origine de réplication virale.

Les composés chimiques ont été évalués pour leur activité inhibitrice de la formation de l'interaction entre les protéines E1 et E2 de VPH11 / VPH6 dans les tests cellulaires en co-transfectant, dans des lignées de cellules humaines dérivées de cellules épithéliales de rein ou de carcinome cervical, le vecteur rapporteur contenant des sites de fixation pour E2 et des couples de vecteurs d'expression des protéines de VPH11 / VPH6, soit d'une part E1 fusionné à VP16 et d'autre part E2. Des doses variées des composés ont été incubées durant 1 à 4 jours après la transfection dans le milieu cellulaire et l'activité luciférase a été déterminée à l'aide d'un luminomètre afin d'évaluer l'IC₅₀ des composés sur la formation de l'interaction entre les protéines E1 et E2 des VPH.

Les composés chimiques 1 et 4 du tableau 1 ont aussi été évalués pour leur activité inhibitrice de la réplication virale dépendante de E1 et E2 de VPH11 / VPH6 dans ces tests cellulaires en co-transfectant, dans des lignées de cellules humaines dérivées de cellules épithéliales de rein ou de carcinome cervical, le vecteur rapporteur-réplicon et des couples de vecteurs d'expression de E1 et E2 de VPH11 / VPH6. Des doses variées des composés ont été incubées durant 2 à 6 jours après la transfection dans le milieu cellulaire et l'activité luciférase a été déterminée à l'aide d'un luminomètre afin d'évaluer l'IC₅₀ des composés sur la réplication du génome des VPH.

Tous les composés présentés dans les exemples ci-dessus inhibent la formation de l'interaction entre les protéines E1 et E2 de VPH11 / VPH6 dans les cellules avec une IC₅₀ inférieure à 20 µM, et pour les composés préférés, inférieure à 10 µM. Ceux évalués dans les tests de réplication virale inhibent la réplication dépendante de E1 et E2 de VPH11 / VPH6 dans les cellules avec une IC₅₀ inférieure à 20 µM, voire inférieure à 10 µM pour les composés les plus actifs.

## Revendications

1. Composés de formule (I) : 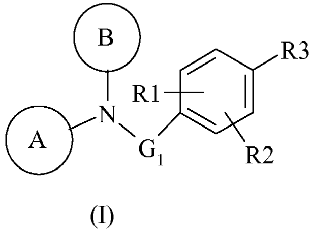 ainsi que leurs stéréoisomères et leurs sels pharmaceutiquement acceptables, dans laquelle :
**G₁** représente un groupement 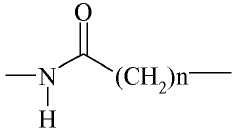 où n est un entier compris entre 1 et 4,
**R1** représente un groupement alkoxy, tel que méthoxy,
**R2** représente un atome d'halogène, tel que le brome,
**R3** représente :
- un groupement : 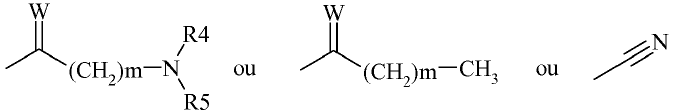 dans lequel :
• **W** représente un atome d'oxygène ou NH,
• **m** est un entier compris entre 0 et 2, et
• **R4** et **R5** représentent, indépendamment l'un de l'autre, un atome d'hydrogène, un groupement alkyle linéaire ou ramifié, tel qu'un méthyle ou un tertiobutyle, ou un groupement alkoxy, tel qu'un méthoxy,
- ou le groupement : 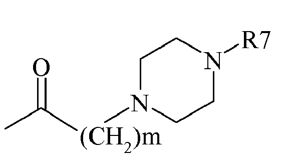 dans lequel R7 représente un groupe alkyle, tel que méthyle, et m représente un entier compris entre 0 et 2,
**A** représente un groupement aryle, tel que phényle, substitué par un groupement alkoxy ou acyle, et
**B** représente un groupement aryle, de préférence un phényle :
• substitué en position ortho par un hétérocycle, et/ou
• substitué en position ortho' par un groupement alkyle.

2. Composés selon la revendication 1, **caractérisés en ce que G₁** représente un groupement 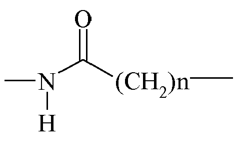 où n vaut 1.

3. Composés selon la revendication 1 ou 2, **caractérisés en ce que R1** représente un groupement alkoxy, tel que méthoxy, situé en position ortho par rapport à R3.

4. Composés selon l'une quelconque des revendications 1 à 3, **caractérisés en ce que R2** représente un atome d'halogène, tel que le brome, situé en position méta par rapport à R3.

5. Composés selon l'une quelconque des revendications 1 à 4, **caractérisés en ce que R3** représente :
- un groupement : 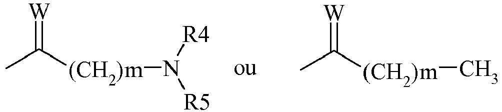 dans lequel :
• **W** représente un atome d'oxygène ou NH,
• **m** est un entier compris entre 0 et 2, et
• **R4** et **R5** représentent, indépendamment l'un de l'autre, un atome d'hydrogène, un groupement alkyle linéaire ou ramifié, tel qu'un méthyle ou un tertiobutyle, ou un groupement alkoxy, tel qu'un méthoxy,
- ou le groupement : 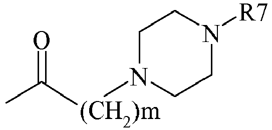 dans lequel R7 représente un groupe alkyle, tel que méthyle, et m représente un entier compris entre 0 et 2.

6. Composés selon l'une quelconque des revendications 1 à 5, **caractérisés en ce que A** représente un groupement aryle, tel que phényle, substitué en position para par un groupement alkoxy ou acyle.

7. Composés selon l'une quelconque des revendications 1 à 6, **caractérisés en ce que B** représente un groupement aryle, de préférence un phényle :
• substitué en position ortho par un N-cycloalkyle, tel qu'un groupement pipéridine, et/ou
• substitué en position ortho' par un groupement alkyle, tel qu'un méthyle.

8. Composés selon l'une quelconque des revendications 1 à 7, choisis dans le groupe suivant :
1) 5-Bromo-2-méthoxy-4-[*N*'-(4-méthoxy-phényl)-*N*'-(2-méthyl-6-pipéridin-1-yl-phényl)-hydrazinocarbonylméthyl]-benzamide ;
2) 5-Bromo-2-méthoxy-4-[*N*'-(4-méthoxy-phényl)-*N'*-(2-méthyl-6-pipéridin-1-yl-phényl)-hydrazinocarbonylméthyl]-*N,N'-*diméthyl-benzamide ;
3) [2-Bromo-5-méthoxy-4-(4-méthyl-pipérazine-1-carbonyl)-phényl]-acétic acid *N'*-(4-méthoxy-phényl)-*N'*-(2-méthyl-6-pipéridin-1-yl-phényl)-hydrazide ;
4) 4-[*N'*-(4-Acétyl-phényl)-*N'*-(2-méthyl-6-pipéridin-1-yl-phényl)-hydrazino carbonylméthyl]-5-bromo-2-méthoxy-benzamide ;
5) (4-Acétyl-2-bromo-5-méthoxy-phényl)-acétic acid *N*'-(4-méthoxy-phényl)-*N*'-(2-méthyl-6-pipéridin-1 -yl-phényl)-hydrazide ;
6) 5-Bromo-2-*N*-diméthoxy-4-[*N*'-(4-méthoxy-phényl)-*N*'-(2-méthyl-6-pipéridin-1-yl-phényl)-hydrazinocarbonylméthyl]-*N*-méthyl-benzamide ;
7) 5-Bromo-*N*-*tert*-butyl-2-méthoxy-4-[*N*'-(4-méthoxy-phényl)-*N*'-(2-méthyl-6-pipéridin-1-yl-phényl)-hydrazinocarbonylméthyl]-benzamide ;
8) (2-Bromo-4-cyano-5-méthoxy-phényl)-acétic acid *N'*-(4-méthoxy-phényl)-*N'*-(2-méthyl-6-pipéridin-1-yl-phényl)-hydrazide.

9. Composition pharmaceutique comprenant au moins un composé de formule (I) selon l'une quelconque des revendications 1 à 8 ou un sel pharmaceutiquement acceptable de celui-ci, en association avec un excipient pharmaceutiquement acceptable.

10. Composé de formule (I) selon l'une quelconque des revendications 1 à 8 ou un sel pharmaceutiquement acceptable de celui-ci pour son utilisation comme médicament.

11. Utilisation d'un composé de formule (I) selon l'une quelconque des revendications 1 à 8 ou d'un sel pharmaceutiquement acceptable de celui-ci pour la préparation d'un médicament destiné à traiter ou prévenir une infection par le virus du papillome.

12. Utilisation selon la revendication 11, pour le traitement ou la prévention des lésions et maladies associées aux infections par le virus du papillome.

13. Utilisation selon la revendication 11 ou 12, pour le traitement ou la prévention des verrues ano-génitales, comme les condylomes acuminés et condylomes plans, les papillomes laryngés, conjonctivaux ou buccaux et d'autres lésions épithéliales, comme des papillomatoses récurrentes respiratoires et des néoplasies intra-épithéliales de bas grade et de haut grade, des papuloses bowénoïdes, des verrues telles que les verrues vulgaires, plantaires, myrmécies, superficielles ou plates, des épidermodysplasies verruciformes, des carcinomes, en particulier ano-génitaux, et toutes les lésions qui sont associées au virus du papillome.

## Patentansprüche

1. Verbindungen der Formel (I): 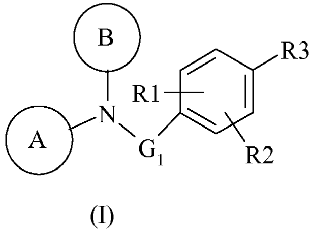 sowie ihre Stereoisomere und ihre pharmazeutisch akzeptablen Salze,
wobei:
**G₁** eine Gruppe 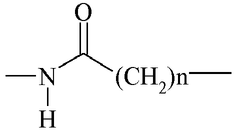 darstellt, wobei n eine Ganzzahl zwischen 1 und 4 inklusive ist,
**R1** eine Alkoxygruppe wie Methoxy darstellt,
**R2** ein Halogenatom wie Brom darstellt,
**R3** darstellt:
- eine Gruppe: 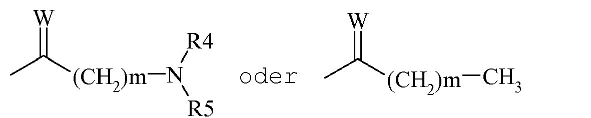 oder 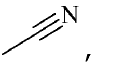 wobei:
• **W** ein Sauerstoffatom oder NH darstellt,
• **m** eine Ganzzahl zwischen 0 und 2 inklusive ist, und
• **R4** und **R5** unabhängig voneinander ein Wasserstoffatom, eine lineare oder verzweigte Alkylgruppe wie ein Methyl oder ein Tertiobutyl oder eine Alkoxygruppe wie ein Methoxy darstellen,
- oder die Gruppe: 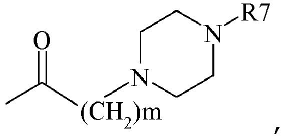 in welcher R7 eine Alklygruppe wie Methyl darstellt und m eine Ganzzahl zwischen 0 und 2 inklusive darstellt,
**A** eine Arylgruppe wie Phenyl, substituiert durch eine Alkoxy- oder Acylgruppe, darstellt, und
**B** eine Arylgruppe, vorzugsweise ein Phenyl, darstellt:
• substituiert an Ortho-Position durch eine Heterocyclus, und/oder
• substituiert an Ortho'-Position durch eine Alkylgruppe.

2. Verbindungen nach Anspruch 1, **dadurch gekennzeichnet, dass G₁** eine Gruppe 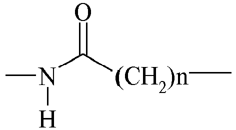 darstellt, wobei n 1 entspricht.

3. Verbindungen nach Anspruch 1 oder 2, **dadurch gekennzeichnet, dass R1** eine Alkoxygruppe wie Methoxy an Ortho-Position im Verhältnis zu R3 darstellt.

4. Verbindungen nach einem der Ansprüche 1 bis 3, **dadurch gekennzeichnet, dass R2** eine Halogenatom wie Brom an Meta-Position im Verhältnis zu R3 darstellt.

5. Verbindungen nach einem der Ansprüche 1 bis 4, **dadurch gekennzeichnet, dass R3** darstellt:
- eine Gruppe 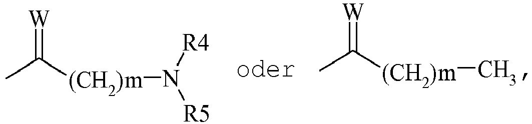 wobei:
• **W** ein Sauerstoffatom oder NH darstellt,
• **m** eine Ganzzahl zwischen 0 und 2 inklusive ist, und
• **R4** und **R5** unabhängig voneinander ein Wasserstoffatom, eine lineare oder verzweigte Alkylgruppe wie ein Methyl oder ein Tertiobutyl, oder eine Alkoxygruppe wie ein Methoxy darstellen,
- oder die Gruppe: 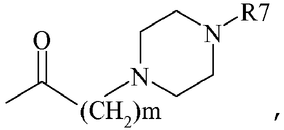 in welcher R7 eine Alklygruppe wie Methyl darstellt und m eine Ganzzahl zwischen 0 und 2 inklusive darstellt.

6. Verbindungen nach einem der Ansprüche 1 bis 5, **dadurch gekennzeichnet, dass A** eine Arylgruppe wie Phenyl, substituiert an Para-Position durch eine Alkoxy- oder Acylgruppe, darstellt.

7. Verbindungen nach einem der Ansprüche 1 bis 6, **dadurch gekennzeichnet, dass B** eine Arylgruppe, vorzugsweise ein Phenyl, darstellt:
• substituiert an Ortho-Position durch ein N-Cycloalkyl wie eine Piperidingruppe, und/oder
• substituiert an Ortho'-Position durch eine Alkylgruppe wie ein Methyl.

8. Verbindungen nach einem der Ansprüche 1 bis 7, ausgewählt aus der folgenden Gruppe:
1) 5-Bromo-2-methoxy-4-[*N*'-(4-methoxy-phenyl)-*N*'-(2-methyl-6-piperidin-1-yl-phenyl)-hydrazincarbonylmethyl]-benzamide,
2) 5-Bromo-2-methoxy-4-[*N*'-(4-methoxy-phenyl)-*N*'-(2-methyl-6-piperidin-1-yl-phenyl)-hydrazincarbonylmethyl]-*N,N'*-dimethyl-benzamid,
3) [2-Bromo-5-methoxy-4-(4-methyl-piperazin-1-carbonyl)-phenyl]-acetic Säure *N*'-(4-methoxy-phenyl)-*N*'-(2-methyl-6-piperidin-1-yl-phenyl)-hydrazid,
4) 4-[*N*'-(4-Acetyl-phenyl)-*N*'-(2-methyl-6-piperidin-1-yl-phenyl)-hydrazincarbonylmethyl]-5-bromo-2-methoxybenzamid,
5) (4-Acetyl-2-bromo-5-methoxy-phenyl)-acetic Säure *N'-*(4-methoxy-phenyl)-*N*'-(2-methyl-6-piperidin-1-yl-phenyl)-hydrazid,
6) 5-Bromo-2-*N*-dimethoxy-4-[*N*'-(4-methoxy-phenyl)-*N*'-(2-methyl-6-piperidin-1-yl-phenyl)-hydrazincarbonylmethyl]-*N*-methyl-benzamid,
7) 5-Bromo-*N*-tert-butyl-2-methoxy-4-[*N*'-(4-methoxyphenyl)-*N*'-(2-methyl-6-piperidin-1-yl-phenyl)-hydrazincarbonylmethyl]-benzamid,
8) (2-Bromo-4-cyano-5-methoxy-phenyl)-acetic Säure *N*'-(4-methoxy-phenyl)-*N'*-(2-methyl-6-piperidin-1-yl-phenyl)-hydrazid.

9. Pharmazeutische Zusammensetzung, umfassend mindestens eine Verbindung der Formel (I) nach einem der Ansprüche 1 bis 8 oder ein pharmazeutisch akzeptables Salz derselben in Kombination mit einem pharmazeutisch akzeptablen Hilfsstoff.

10. Verbindung der Formel (I) nach einem der Ansprüche 1 bis 8 oder ein pharmazeutisch akzeptables Salz derselben für ihre/seine Verwendung als Arzneimittel.

11. Verwendung einer Verbindung der Formel (I) nach einem der Ansprüche 1 bis 8 oder eines pharmazeutisch akzeptablen Salzes derselben für die Herstellung eines Arzneimittels, das zur Behandlung oder Vorbeugung einer Infektion mit dem Papillomavirus bestimmt ist.

12. Verwendung nach Anspruch 11 zur Behandlung oder Vorbeugung von Läsionen und Krankheiten, die mit Infektionen mit dem Papillomavirus verbunden sind.

13. Verwendung nach Anspruch 11 oder 12 zur Behandlung oder Vorbeugung von Analgenitalwarzen wie die spitzen Kondylome und flachen Kondylome, von Papillomen von Kehlkopf, Bindehaut oder Mundhöhle und anderen Epithelialläsionen, wie die rezidivierenden Atemwegspapillomatosen und die intraepithelialen Neoplasien niederen und höheren Grades, von bowenoiden Papulosen, von Warzen wie die gemeinen, Fußsohlen-, Dorn-, oberflächlichen oder flachen Warzen, von Epidermodysplasia verruciformis, von Karzinomen, insbesondere Anal-Genital-Karzinomen, und von allen Läsionen, die mit dem Papillomavirus verbunden sind.

## Claims

1. Compounds of formula (I): 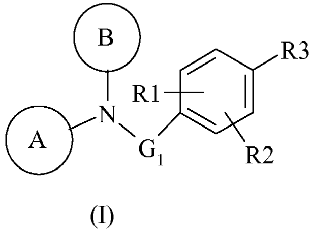 as well as their stereoisomers and their pharmaceutically acceptable salts,
wherein:
**G₁** represents a group 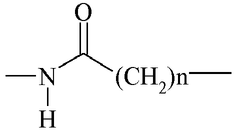 wherein n is an integer comprised between 1 and 4,
**R1** represents an alkoxy group, such as methoxy,
**R2** represents a halogen atom, such as bromine,
**R3** represents:
- a group: 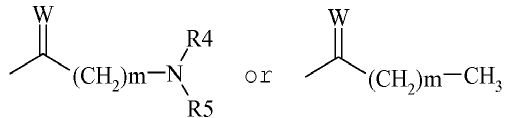 or 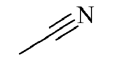 wherein:
• **W** represents an oxygen atom or NH,
• **m** is an integer comprised between 0 and 2, and
• **R4** and **R5** represent independently of each other a hydrogen atom, a linear or branched alkyl group, such as a methyl or tertiobutyl, or an alkoxy group, such as a methoxy,
- or the group: 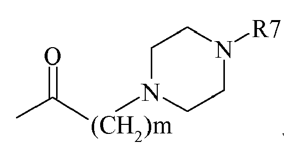 wherein R7 represents an alkyl group, such as methyl, and **m** is an integer comprised between 0 and 2,
**A** represents an aryl group, such as phenyl, substituted with an alkoxy or acyl group, and
**B** represents an aryl group, preferably a phenyl:
• substituted in the ortho position with a heterocycle, and/or
• substituted in the ortho' position with an alkyl group.

2. Compounds according to claim 1, **characterized in that G₁** represents a group 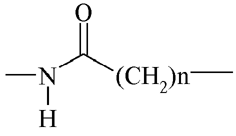 wherein n is 1.

3. Compounds according to claim 1 or 2, **characterized in that R1** represents an alkoxy group, such as methoxy, in the ortho position relatively to R3.

4. Compounds according to any one of claims 1 to 3, **characterized in that R2** represents a halogen atom, such as bromine, in the meta position relatively to R3.

5. Compounds according to any one of claims 1 to 4, **characterized in that R3** represents:
- a group 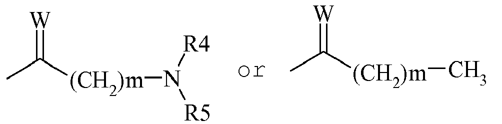 wherein:
• **W** represents an oxygen atom or NH,
• **m** is an integer comprised between 0 and 2, and
• **R4** and **R5** represent independently of each other a hydrogen atom, a linear or branched alkyl group, such as a methyl or tertiobutyl, or an alkoxy group, such as a methoxy,
- or the group: 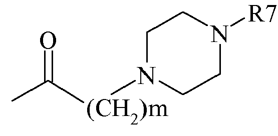 wherein R7 represents an alkyl group, such as methyl, and m is an integer comprised between 0 and 2.

6. Compounds according to any one of claims 1 to 5, **characterized in that A** represents an aryl group, such as phenyl, substituted in the para position with an alkoxy or acyl group.

7. Compounds according to any one of claims 1 to 6, **characterized in that B** represents an aryl group, preferably a phenyl:
• substituted in the ortho position with a N-cycloalkyl, such as a piperidine group, and/or
• substituted in the ortho' position with an alkyl group, such as a methyl.

8. Compounds according to any one of claims 1 to 7, selected from the following group:
1) 5-Bromo-2-methoxy-4-[*N*'-(4-methoxy-phenyl)-*N*'-(2-methyl-6-piperidin-1-yl-phenyl)-hydrazinocarbonylmethyl]-benzamide;
2) 5-Bromo-2-methoxy-4-[*N*'-(4-methoxy-phenyl)-*N*'-(2-methyl-6-piperidin-1-yl-phenyl)-hydrazinocarbonylmethyl]-N,N-dimethyl-benzamide;
3) [2-Bromo-5-methoxy-4-(4-methyl-piperazine-1-carbonyl)-phenyl]-acetic acid N'-(4-methoxy-phenyl)-*N'-*(2-methyl-6-piperidin-1-yl-phenyl)-hydrazide;
4) 4-[*N*'-(4-Acetyl-phenyl)-*N*'-(2-methyl-6-piperidin-1-yl-phenyl)-hydrazino-carbonylmethyl]-5-bromo-2-methoxybenzamide;
5) (4-Acetyl-2-bromo-5-methoxy-phenyl)-acetic acid *N'-*(4-methoxy-phenyl)-*N*'-(2-methyl-6-piperidin-l-yl-phenyl)-hydrazide;
6) 5-Bromo-2-N-dimethoxy-4-[*N*'-(4-methoxy-phenyl)-N'-(2-methyl-6-piperidin-1-yl-phenyl)-hydrazinocarbonylmethyl]-*N-*methyl-benzamide;
7) 5-Bromo-*N*-*tert*-butyl-2-methoxy-4-[*N*'-(4-methoxy-phenyl)-*N*'-(2-methyl-6-piperidin-1-yl-phenyl)-hydrazinocarbonyl-methyl]-benzamide;
8) (2-Bromo-4-cyano-5-methoxy-phenyl)-acetic acid *N*'-(4-methoxy-phenyl)-*N*'-(2-methyl-6-piperidin-1-yl-phenyl)-hydrazide.

9. A pharmaceutical composition comprising at least one compound of formula (I) according to any one of claims 1 to 8 or a pharmaceutically acceptable salt thereof, in association with a pharmaceutically acceptable excipient.

10. A compound of formula (I) according to any one of claims 1 to 8 or a pharmaceutically acceptable salt thereof for its use as a drug.

11. The use of a compound of formula (I) according to any one of claims 1 to 8 or of a pharmaceutically acceptable salt thereof for the preparation of a drug intended to treat or prevent an infection by the papilloma virus.

12. The use according to claim 11, for treating or preventing lesions and diseases associated with infections by the papilloma virus.

13. The use according to claim 11 or 12, for treating or preventing ano-genital verrucas such as acuminated condylomas and planar condylomas, laryngeal, conjunctival or buccal papillomas and other epithelial lesions such as recurrent respiratory papillomatoses and low grade and high grade intra-epithelial neoplasias, bowenoid papuloses, verrucas such as vulgar, plantar, myrmecias, superficial or flat verrucas, verruciform epidermaldysplasias, carcinomas, in particular ano-genital carcinomas, and all the lesions which are associated with the papilloma virus.
